# EUROPÄISCHE PATENTSCHRIFT

(11) **EP 1 949 097 B1**
(45) Veröffentlichungstag und Bekanntmachung des Hinweises auf die Patenterteilung: **06.06.2012**
(21) Anmeldenummer: 06817713.8
(22) Anmeldetag: 15.11.2006
(51) Int. Cl.: G01N 33/36, G01N 21/89

(54) **Verfahren zur Charakterisierung von Effektgarn**
Method for characterising effect yarn
Procédé pour caractériser un filé a effets

(30) Priorität: 18.11.2005 CH 18462005; 23.12.2005 CH 20592005; 08.06.2006 CH 9252006; 21.06.2006 CH 10042006
(43) Veröffentlichungstag der Anmeldung: 30.07.2008
(60) Teilanmeldung: 10012544.2
(73) Patentinhaber: Uster Technologies AG, 8610 Uster (CH)
(72) Erfinder: MEIXNER, Christine, CH-8636 Wald (CH); PETERS, Gabriela, CH-8320 Fehrlatorf (CH); EDALAT-POUR, Sandra, CH-8340 Hadlikon (CH)
(86) Internationale Anmeldenummer: PCT/CH2006/000644
(87) Internationale Veröffentlichungsnummer: WO 2007/056885

(56) Entgegenhaltungen:
- EP-B1- 0 892 925
- DE-A1- 3 237 371
- DE-A1- 4 413 549
- DE-A1- 10 348 742
- DE-A1- 19 535 177

## Beschreibung

### FACHGEBIET

Die Erfindung betrifft ein Verfahren zur Charakterisierung von Effektgarn, gemäss Oberbegriff des ersten Patentanspruchs. Sie kann sowohl im Textillabor (offline) als auch in der Textilproduktion (online), z. B. in Spinnerei oder Spulerei, eingesetzt werden.

### STAND DER TECHNIK

Effektgarn ist Garn, dessen Struktur oder Faserzusammensetzung von normalem Glattgarn abweicht. Es wird als bereicherndes Element in Weberei- und Strickereiprodukten eingesetzt. Üblicherweise weist Effektgarn eine Vielzahl von Dickstellen oder Dünnstellen - so genannten Effekten - auf, deren Durchmesser deutlich grösser bzw. kleiner ist als der Durchmesser der zwischen zwei Effekten liegenden Garnabschnitte - der so genannten Stege. Aber auch strukturiertes Garn mit absichtlich erzeugten Dickenvariationen, bei dem keine Stege identifiziert werden können, wird zum Effektgarn gezählt. Die zunehmende Beliebtheit von Effektgarn verlangt nach zuverlässigen und aussagekräftigen Verfahren zu dessen Charakterisierung. Insbesondere interessieren Grössen wie Stegdurchmesser, Durchmesserzunahme bei einem Effekt, Effektmasse, Effektlänge, Steglänge etc. Diese Grössen können z. B. zur Kontrolle der Qualität eines vorliegenden Effektgarns oder zur Bestimmung der Herstellungsparameter, die zum Kopieren eines gegebenen Effektgarns nötig sind, verwendet werden.

Verfahren und Vorrichtungen zur Charakterisierung von Garn sind bekannt. Sie basieren üblicherweise auf einer kapazitiven und/oder optischen Abtastung des in Längsrichtung bewegten Garns. Das kapazitive Abtastprinzip liefert ein der Garnmasse entsprechendes Signal, während das optische Abtastprinzip ein zum Garndurchmesser proportionales Signal liefert. Das Abtastsignal wird analog und/oder digital ausgewertet, und ein oder mehrere Resultate der Auswertung wird bzw. werden ausgegeben. Beispiele für derartige Verfahren und Vorrichtungen zur Charakterisierung von Garn sind in den Patentveröffentlichungen EP-0'578'975 A1 oder EP-0'249'741 A2 angegeben; beide beziehen sich auf das Garnprüfsystem USTER^{®} TESTER, das weltweit von der Inhaberin des vorliegenden Schutzrechtes vertrieben wird.

Es ist auch bekannt, Information über die Farbe, d. h. über die spektralen Reflexionseigenschaften, von Garn zu gewinnen. So wird z. B. gemäss der WO-2004/044579 eine mehrfarbige Lichtquelle zur Beleuchtung des Garns verwendet. Das vom Garn reflektierte Licht wird in mindestens zwei verschiedenen Spektralbereichen getrennt detektiert. Die mindestens zwei Detektionssignale erlauben eine Aussage über die Garnfarbe. Eine gleichzeitige optische Abtastung eines textilen Materials mit verschiedenen Wellenlängen ist auch aus der CH 674'379 oder aus der DE-198'59'274 bekannt.

Speziell mit Effektgarn befassen sich die WO-2005/071'150 A1, die WO-2005/038'105 A1 und die WO-2005/037699 A1. Die Lehre der Letzteren kann wie folgt zusammengefasst werden:
- Bestimmung des Stegdurchmessers: Zunächst wird der arithmetische Mittelwert des Garndurchmessers über eine grosse Garnlänge gebildet. Dieser Mittelwert wird von den Einzelwerten des Garndurchmessers subtrahiert. Als Stegdurchmesser wird der arithmetische Mittelwert aus allen negativen Werten, die benachbart zu anderen negativen Werten gemessen wurden, definiert.
- Bestimmung des Beginns, des Endes und der Länge eines Effektes: Ein Effektbeginn liegt dann vor, wenn ein Grenzdurchmesser überschritten wird, der über dem Stegdurchmesser liegt, und das Überschreiten über eine gewisse Garnlänge andauert. Ein Effektende liegt dann vor, wenn der Grenzdurchmesser unterschritten wird und das Unterschreiten über eine gewisse Garnlänge andauert. Die Effektlänge wird als Distanz zwischen Effektbeginn und Effektende definiert.
- Bestimmung des Effektdurchmessers: Innerhalb eines Effektes wird eine Mehrzahl grösster Durchmesser ermittelt. Der Effektdurchmesser wird als Mittelwert dieser grössten Durchmesser definiert.
Obwohl diese Lehre eine brauchbare Charakterisierung von Effektgarn ermöglicht, hat sie auch einige Nachteile. Als Resultat fällt eine grosse Datenmenge an, die unübersichtlich und schwer handhabbar ist. Geeignete Möglichkeiten zur übersichtlichen Darstellung der Messresultate werden nicht angegeben. Das beschriebene Verfahren liefert einen eher zu grossen Stegdurchmesser und eine eher zu kleine Effektlänge.

Die DE-195'35'177 A1 offenbart ein Verfahren und eine Vorrichtung zum Prüfen der Struktur von Garnen, unter Anderem auch von Effektgarnen. Es können verschiedene Abtastorgane zur Abtastung des Garns eingesetzt werden, so z. B. ein mit vorbestimmter Gleichspannung aufgeladener U-förmiger Kondensator, dessen innere Elektrode mit dem Garn in Kontakt steht. Wird ein elektrostatisch aufgeladenes Garn hindurchbewegt, wird der Ruheladung des Kondensators eine rauschartige Wechselladung überlagert. Ein an der inneren Elektrode abgegriffenes Messsignal wird durch Tiefpass- oder Bandpassfilterung geglättet. Anhand des geglätteten Signals können mit Hilfe eines Schwellwertentscheiders die Strukturdetails des Garns in Echtzeit überwacht werden.

In der DE-32'37'371 A1 sind ein Verfahren und eine Vorrichtung zur Prüfung der Garngleichmässigkeit bei einer Spinnmaschine beschrieben. Die Garndicke wird optisch gemessen, das analoge Messsignal wird digitalisiert und zur Analyse zyklischer und nicht zyklischer Garnungleichmässigkeiten in einem Rechner ausgewertet. Die Auswertung kann z. B. mittels einer Spektralanalyse und einer Integralanalyse erfolgen. Bei der Spektralanalyse wird das digitalisierte Messsignal Fourier-transformiert und als Spektrum ausgegeben. Das Spektrum erlaubt eine Beurteilung der zyklischen Garnunregelmässigkeiten. Bei der Integralanalyse wird der Absolutwert der Abweichung von einem festgelegten Wert über eine bestimmte Garnlänge integriert. Je grösser der Integralwert, umso grösser ist die nicht zyklische Garnungleichmässigkeit.

Ein Verfahren zur Herstellung oder Bearbeitung eines laufenden Garns, das zu einer Kreuzspule aufgewickelt wird, ist in der DE-44'13'549 A1 offenbart. In der Aufwickelzone wird die Fadenzugkraft gemessen. Das Ausgangssignal wird mit einem Referenzsignal verglichen. Das Referenzsignal beinhaltet den hypothetischen Fadenzugkraftverlauf, der durch Änderung der Changiergeschwindigkeit entsteht. Der hypothetische Fadenzugkraftverlauf kann z. B. gewonnen werden, indem der Fadenzugkraftverlauf einer Spulreise gespeichert wird, die sich durch optimale Einstellung und störungsfreien Verlauf auszeichnet. Dieser Fadenzugkraflverlauf wird vor dem Speichern noch "idealisiert", indem er geglättet und von zufallsbedingten Störeinflüssen bereinigt wird. Das Ergebnis des Vergleiches ist ein bereinigter Fadenzugkraftverlauf, der Aufschluss gibt über Störungen des Herstellungs- bzw. Bearbeitungsverfahrens und die Qualität der Spule.

Die EP-0'892'925 B1 offenbart ein Verfahren und eine Vorrichtung zum Nachweis untexturierter bzw. fehlerhaft texturierter Garnabschnitte in texturierten Filamentgarnen während des Texturierprozesses. Das texturierte Garn wird laufend gemessen, indem hochfrequente Messsignale als Nachweise für untexturierte bzw. fehlerhaft texturierte Garnabschnitte gemessen, aufbereitet und ausgewertet werden. Die Auswertung kann eine Glättung oder Filterung der Messsignale beinhalten. Die Anzahl der einen vorgegebenen Schwellenwert überschreitenden hochfrequenten Fadenzugkraftsignale gibt Aufschluss über die Existenz von kurzen Störstellen im Garn.

### DARSTELLUNG DER ERFINDUNG

Es ist eine Aufgabe der vorliegenden Erfindung, ein Verfahren zur Charakterisierung von Effektgarn anzugeben, das die Menge der anfallenden Daten reduziert. Information über die Effekte und das Grundgarn soll getrennt erhalten und ausgegeben werden können. Ferner soll die Erfindung eine bessere visuelle Erfassung der Charakteristika des Effektgarns erlauben.

Diese und andere Aufgaben werden durch das in Anspruch 1 definierte Verfahren gelöst. Vorteilhafte Ausführungsformen sind in den abhängigen Patentansprüchen angegeben.

Gemäss dem erfindungsgemässen Verfahren zur Charakterisierung von Effektgarn, das vorzugsweise eine Abfolge von Effekten und Stegen aufweist, wird mindestens eine Eigenschaft des Effektgarns entlang der Längsrichtung des Effektgarns abgetastet. Es werden Werte der Abtastung ausgewertet und Resultate der Auswertung ausgegeben; es handelt sich vorzugsweise um Effektgarnparameter wie Stegmasse, Stegdurchmesser, Steglänge, Massenzunahme eines Effektes, Effektdurchmesserzunahme, Effektdurchmesser, Effektlänge und/oder Effektmasse. Die Auswertung beinhaltet eine Glättung oder Idealisierung der Abtastwerte. In einer bevorzugten Ausführungsform beinhaltet die Auswertung eine Verknüpfung der geglätteten oder idealisierten Abtastwerte mit den ursprünglichen Abtastwerten, z. B. eine Differenzbildung.

Die geglätteten oder idealisierten Abtastwerte werden vorzugsweise separat ausgewertet, um Information über die Effekte einerseits bzw. über das Grundgarn andererseits zu erhalten. Durch Angabe von Parametern des idealisierten Kurvenverlaufs kann die anfallende Datenmenge reduziert werden.

### AUFZÄHLUNG DER ZEICHNUNGEN

Nachfolgend wird die Erfindung anhand der Zeichnungen detailliert erläutert.
- Figur 1: zeigt schematisch eine Vorrichtung zur Durchführung des erfindungsgemässen Verfahrens.
- Figur 2: zeigt ein Beispiel einer Reihe von Messwerten an einem Effektgarn, nämlich Garnmasse versus Längskoordinate.
- Figur 3: zeigt eine Häufigkeitsverteilung der gemessenen, in Figur 2 aufgetragenen Garnmasse.
- Figur 4: zeigt einen vergrösserten Ausschnitt aus Figur 2.
- Figuren 5-15: zeigen mögliche erfindungsgemässe Darstellungsarten für die Ausgabe der Garnparameter.
- Figur 16: zeigt ein Beispiel einer Reihe von Messwerten an einem zweistufigen Effektgarn, in derselben Darstellung wie Fig. 2.
- Figur 17: zeigt eine mögliche erfindungsgemässe Darstellungsart für die Ausgabe der Charakteristika eines mehrstufigen Garns.
- Figur 18: zeigt ein Spektrogramm einer Reihe von Messwerten an einem Effektgarn.
- Figur 19: zeigt einen gemessenen und einen idealisierten Effekt.
- Figur 20: zeigt eine zur Figur 4 analoge Messkurve mit einer Dünnstelle.
- Figur 21: zeigt den Verlauf der Fläche unter einer Messkurve als Funktion der Längskoordinate.
- Figur 22: zeigt die Masse pro Längeneinheit als Funktion der Längskoordinate (a) für die Messkurve, (b) für eine idealisierte Kurve und (c) für eine Kurve, die durch Subtraktion der Kurve (b) von der Kurve (a) entsteht.
- Figur 23: zeigt Spektrogramme (a) der Kurve von Fig. 22(a), (b) der Kurve von Fig. 22(b) und (c) der Kurve von Fig. 22(c).
- Figur 24: zeigt Scatterplots (a) der Messwerte von Fig. 22(a), (b) der Effekte von Fig. 22(b) und (c) des Grundgarns von Fig. 22(c).

### AUSFÜHRUNG DER ERFINDUNG

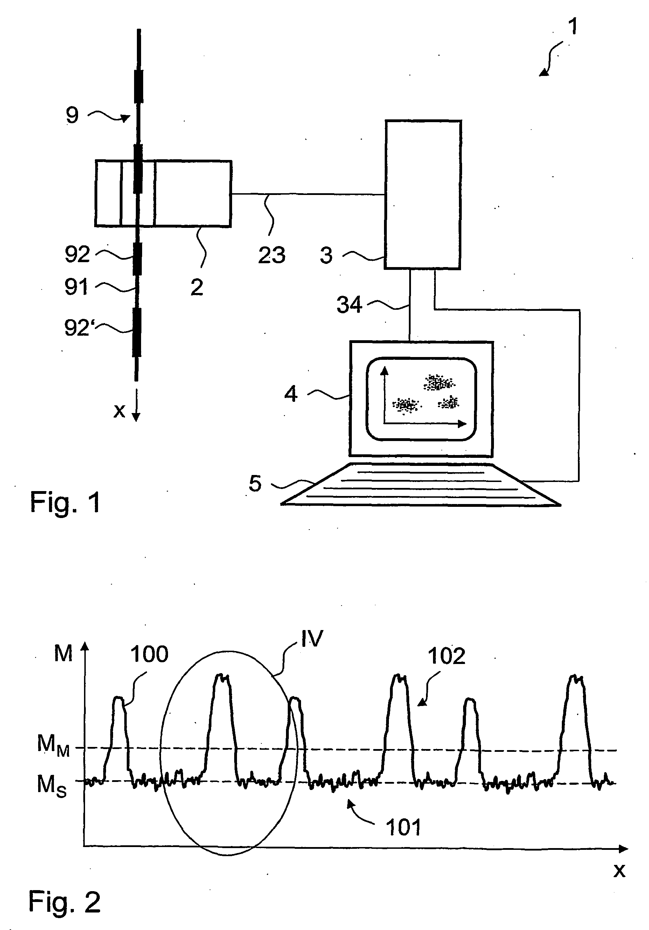

In Figur 1 ist schematisch eine Vorrichtung 1 zur Durchführung des erfindungsgemässen Verfahrens dargestellt. Sie beinhaltet eine Abtasteinheit 2 zum Abtasten eines in Längsrichtung -x bewegten Effektgarns 9 mit Stegen 91 und Effekten 92, 92'. Unter dem Begriff "Abtasten" wird hier die sequenzielle Aufnahme einer Vielzahl von Messwerten an verschiedenen, vorzugsweise äquidistanten Stellen des Effektgarns 9 verstanden. Derartige Abtasteinheiten 2 sind an sich bekannt und brauchen hier nicht näher erläutert zu werden. Die Abtasteinheit 2 kann einen kapazitiven, optischen oder anderen Sensor beinhalten; es können auch mehrere gleiche oder verschiedene Sensoren innerhalb der Abtasteinheit 2 angeordnet sein. Die Abtasteinheit 2 kann mit Auswertemitteln für eine Vorauswertung der Messdaten ausgestattet sein. Sie gibt auf einer ersten Datenleitung 23 ein vorzugsweise elektrisches Ausgangssignal aus, das ein Mass ist für die Masse, die Dicke oder andere Eigenschaften des Effektgarns 9.

Die erste Datenleitung 23 mündet in eine Auswerteeinheit 3, die zum Auswerten des Ausgangssignals der Abtasteinheit 2 geeignet ist. Zu diesem Zweck beinhaltet sie geeignete analoge und/oder digitale Auswertemittel, z. B. einen Mikroprozessor. Sie kann auch weitere Mittel wie Speichermittel zum Speichern von Daten beinhalten. Die Auswerteeinheit 3 ist vorzugsweise ein Computer.

Ferner beinhaltet die Vorrichtung 1 eine Ausgabeeinheit 4 zum Ausgeben von Messdaten und/oder Resultaten der Auswertung. Die Ausgabeeinheit 4 ist mittels einer zweiten Datenleitung 34 mit der Auswerteeinheit 3 verbunden. Sie kann z. B. als Bildschirm und/oder Drucker ausgebildet sein. Vorzugsweise beinhaltet die Vorrichtung 1 auch eine Eingabeeinheit 5 zum Eingeben von Daten durch einen Benutzer. Die Eingabeeinheit 5 kann z. B. eine Tastatur, eine Maus und/oder einen Touch-Screen beinhalten.

Figur 2 zeigt ein mögliches Ausgangssignal 100 der Abtasteinheit 2. Dabei ist bspw. eine Grösse, die ein Mass für die Masse M pro Längeneinheit des Effektgarns 9 (siehe Fig. 1) ist, gegenüber der Längskoordinate x des Effektgarns 9 aufgetragen; ein solches Massensignal M wird typischerweise von einem kapazitiven Garnsensor geliefert. Ähnlich würde eine Darstellung der Dicke des Effektgarns 9 gegenüber der Längskoordinate x aussehen, wobei die Massstäbe auf der Abszisse und der Ordinate sowie die Auflösung verschieden sein könnten; ein Dickensignal wird typischerweise von einem optischen Garnsensor geliefert. Die Kurve M(x) ist nicht notwendigerweise kontinuierlich, sondern kann aus einzelnen (in Fig. 2 nicht dargestellten) Abtastpunkten zusammengesetzt sein, die auf dem Effektgarn 9 typischerweise um einige Millimeter voneinander entfernt sind; die Entfernung hängt von der Abtastrate der Abtasteinheit 2 und von der Geschwindigkeit des Effektgarns 9 ab. Das Signal M(x) hat einen Grundpegel 101, welcher der Stegmasse Ms bzw. dem Stegdurchmesser entspricht. Aus dem Grundpegel 101 ragen deutlich den Effekten entsprechende Spitzen 102 heraus. Ein Mittelwert M_{M} aller Messwerte M(x) über eine grosse Garnlänge liegt wegen der Spitzen 102 deutlich über dem Grundpegel 101 und ist deshalb als Mass für die Stegmasse M_{S} ungeeignet. An dieser Stelle sei bemerkt, dass Figur 2 und die nachfolgende Diskussion nur ein mögliches, nicht einschränkendes Beispiel darstellt. Bei anderen Arten von Effektgarn ist es gar nicht mehr möglich, Stege zu identifizieren.

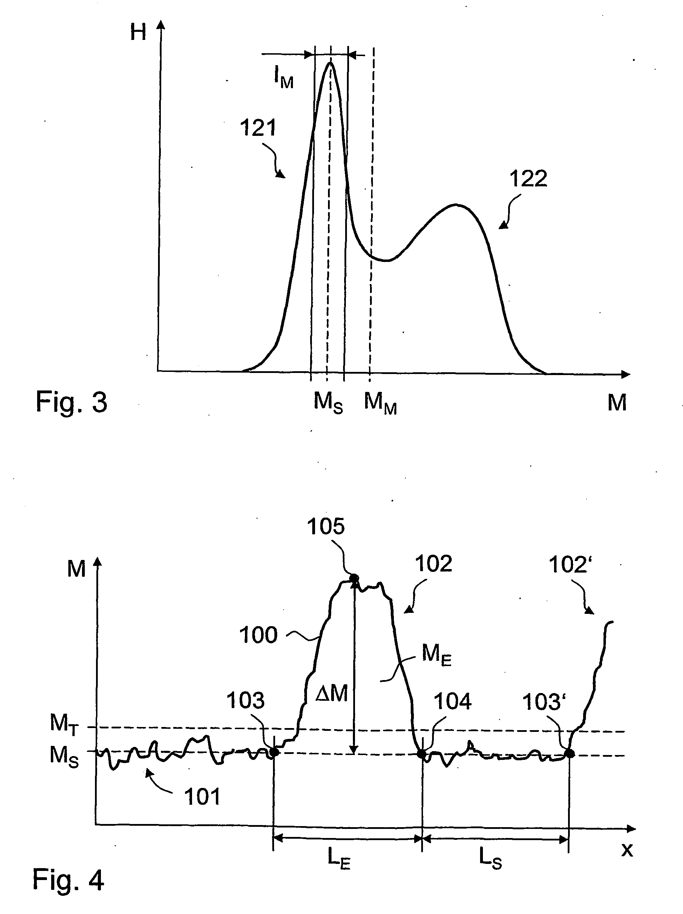

Die Stegmasse M_{S} kann gemäss der vorliegenden Erfindung vorzugsweise wie folgt ermittelt werden. Es wird eine Häufigkeitsverteilung H(M) der gemessenen und in Figur 2 dargestellten Massen M ermittelt. Eine derartige Häufigkeitsverteilung H(M) ist schematisch in Figur 3 gezeigt. Die Häufigkeitsverteilung H(M) muss für eine grosse Garnlänge ermittelt werden, worunter hier eine Garnlänge verstanden wird, die viele, bspw. mindestens 10 und vorzugsweise mindestens 100, Effekte beinhaltet. Die Häufigkeitsverteilung H(M) weist bei einem Effektgarn 9 mindestens zwei lokale Maxima 121, 122 auf: ein erstes lokales Maximum 121 für die Stege 91 und mindestens ein zweites lokales Maximum 122 für die Effekte 92, 92', ... Definitionsgemäss hat unter allen lokalen Maxima 121, 122 das zu den Stegen 91 gehörige lokale Maximum 121 die kleinste Masse M. Als Stegmasse M_{S} wird deshalb die kleinste Masse M definiert, bei welcher in der Häufigkeitsverteilung H(M) ein lokales Maximum 121 auftritt. Aus der Stegmasse M_{S} kann die Garnnummer des Garnkörpers bzw. der Stege 91 berechnet werden. Analog kann vorgegangen werden, um einen Stegdurchmesser zu ermitteln.

Gemäss einer alternativen Ausführungsform des erfindungsgemässen Verfahrens zur Ermittlung der Stegmasse wird ein Massenintervall IM (siehe Figur 3) definiert, welches diejenige kleinste Masse beinhaltet, bei welcher in der Häufigkeitsverteilung (H(M)) ein lokales Maximum 121 auftritt,. Das Massenintervall IM kann, muss aber nicht bezüglich der "kleinsten" Masse symmetrisch sein, d. h. die "kleinste" Masse kann, muss aber nicht in der Mitte des Massenintervalls IM liegen. Die obere Grenze des Massenintervalls IM wird vorzugsweise unterhalb des globalen Massenmittelwertes M_{M} gewählt. Breite und Lage des Massenintervalls I_{M} können fest vorgegeben sein - bspw. ±5 % der "kleinsten" Masse -, automatisch von der Auswerteeinheit 3 berechnet oder durch einen Benutzer eingegeben werden. Anschliessend wird ein Mittelwert über alle in diesem Massenintervall IM gemessenen Massen M gebildet. Die Stegmasse wird als dieser Mittelwert definiert.

Figur 4 zeigt in detaillierterer Ansicht einen Ausschnitt aus der Messkurve M(x) von Fig. 2. Anhand von Figur 4 wird nachfolgend erläutert, wie weitere Effektgarnparameter wie Effektlänge L_{E}, Steglänge L_{S}, Massenzunahme ΔM und Effektgesamtmasse M_{E} ermittelt werden.

Zur Feststellung eines Beginns 103 und eines Endes 104 eines Effektes 92 wird vorgängig ein Schwellenwert M_{T} festgelegt, der grösser ist als die Stegmasse M_{S}. Der Schwellenwert-M_{T} kann fest vorgegeben sein - bspw. 110 % der Stegmasse M_{S} -, automatisch von der Auswerteeinheit 3 berechnet oder durch einen Benutzer eingegeben werden. Der Beginn 103 eines Effektes 92 liegt dann vor, wenn ausgehend vom Grundpegel 101 der Schwellenwert M_{T} überschritten wird. Um Artefakte durch Ausreisser auszuschliessen, kann zusätzlich kontrolliert werden, ob das Überschreiten über eine vorbestimmte Garnlänge andauert, d. h. ob einige weitere Messpunkte, die unmittelbar auf den den Schwellenwert M_{T} überschreitenden Messpunkt folgen, ebenfalls über dem Schwellenwert M_{T} liegen. Zur exakteren Ermittlung des Beginns 103 des Effektes 92 geht man auf der Messkurve M(x) so weit zurück, bis erstmals ein Messwert kleiner oder gleich der Stegmasse M_{S} - oder einem anderen, vordefinierten oder berechneten Wert - ist. Dieser Messwert wird als Beginn 103 des Effektes 92 definiert. In einem analogen Verfahren wird, mutatis mutandis, das Ende 104 des Effektes 92 ermittelt: Unterschreiten des Schwellenwertes M_{T} ausgehend von der Signalspitze 102, auf der Messkurve M(x) so weit vorwärts, bis erstmals ein Messwert kleiner oder gleich der Stegmasse M_{S} ist. Bei Bedarf können zur Feststellung des Effektbeginns 103 und des Effektendes 104 unterschiedliche Schwellenwerte verwendet werden.

Die Effektlänge L_{E} wird gemäss der vorliegenden Erfindung als Entfernung zwischen dem Beginn 103 und dem Ende 104 des Effektes 92 definiert. Die Steglänge L_{S} wird als Entfernung zwischen dem Ende 104 eines Effektes und dem Beginn 103' eines nachfolgenden Effektes 92', zu dem eine nachfolgende Signalspitze 102' gehört, definiert. Die Entfernung zwischen zwei benachbarten Effekten 92, 92' wird als die Summe L_{E} + L_{S} von Effektlänge und Steglänge definiert. Typische Effektlängen L_{E} und Steglängen L_{S} liegen im Bereich zwischen 2 cm und einigen Metern.

Die Massenzunahme ΔM, die ungefähr einer Durchmesserzunahme des Effektgarns 9 entspricht, wird im einfachsten Fall als Differenz zwischen einem lokalen Maximum 105 der entsprechenden Signalspitze 102 und der Stegmasse M_{S} definiert. Zur Ermittlung der Massenzunahme ΔM sind auch verfeinerte Verfahren möglich, die Schwankungen des Abtastsignals M(x) entlang der Effektlänge Rechnung tragen. So könnte zum Beispiel - analog zur oben beschriebenen Ermittlung der Stegmasse M_{S} - der häufigste Wert innerhalb der entsprechenden Effektlänge ausgewählt werden. Alternativ kommt eine Mittelwertbildung von Werten auf dem Effektkamm in Frage. Die Massenzunahme ΔM wird vorzugsweise als Vielfaches der Stegmasse M_{S} angegeben, z. B. in Prozentwerten, wobei die Stegmasse Ms vorzugsweise als 100 % definiert ist. Typische Massenzunahmen ΔM liegen im Bereich zwischen 20 % und 500 %.

Ein weiterer Parameter zur Charakterisierung eines Effektes 92 ist die so genannte Effektgesamtmasse M_{E}. Diese ist im Wesentlichen die Differenz zwischen (i) dem Integral der Messkurve M(x) über die Effektlänge L_{E} und (ii) der Masse M_{S}·L_{E} des Garnkörpers auf dieser Effektlänge L_{E}. Die Effektgesamtmasse M_{E} kann rechnerisch von der Auswerteeinheit 3 ermittelt werden. Aus der Effektgesamtmasse M_{E} kann die Garnnummer des Effektes 92 berechnet werden, wobei die Berechnung eine Division der Effektgesamtmasse M_{E} durch die Effektlänge L_{E} beinhalten kann.

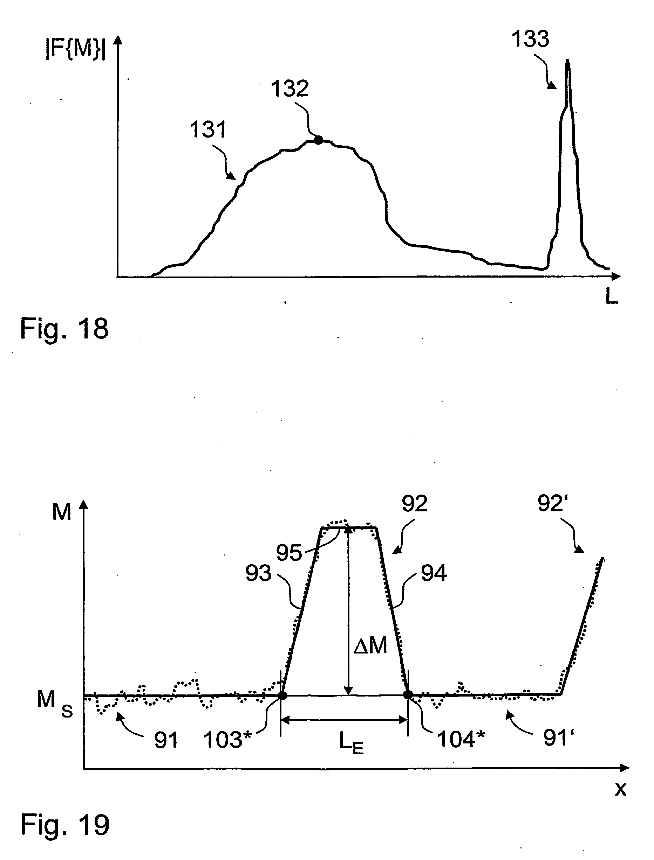

Auch die Form eines Effektes 92 kann ermittelt und ausgegeben werden. Dabei kann auf einen Vergleich mit einfachen geometrischen Formen wie Säule, Dreieck, Treppe, Trapez oder Glocke zurückgegriffen werden; vgl. Fig. 18. Die entsprechende Form kann z. B. auf der Ausgabeeinheit 4 ausgegeben werden.

Es interessieren nicht nur "lokale" Parameter der einzelnen Effekte, sondern auch "globale" Parameter eines ganzen Garnabschnitts, eines Garns oder einer Gruppe von mehreren Garnen. Ein solcher globaler Effektgarnparameter ist die mittlere Garnnummer (mittlere Garnmasse), die sich bspw. durch Mittelwertbildung über alle Messwerte berechnen lässt. Auch die Garnnummer aller Stege 91 kann interessieren. Ein weiterer globaler Effektgarnparameter ist die mittlere räumliche Frequenz der Effekte, d. h. die mittlere Anzahl Effekte pro Längeneinheit.

Die oben genannten und möglicherweise weitere Effektgarnparameter werden vorzugsweise dynamisch, während der Laufzeit der Abtastung, ermittelt. Die Effektparameter werden zwecks Ausgabe gespeichert. Es ist von Vorteil, zusätzlich auch eine durchgehende, dem jeweiligen Effekt zugeordnete Laufnummer zu speichern, um Information nicht nur über die einzelnen Effekte, sondern auch über ihre Reihenfolge zur Verfügung stellen zu können.

Es kann vorteilhaft sein, die Abtasteinheit 2 (Fig. 1) sowohl mit einem kapazitiven als auch mit einem optischen Garnsensor auszustatten, die gleichzeitig ein und dasselbe Garn ausmessen können. Die Ausgangssignale des kapazitiven und des optischen Sensors können zwecks einer besseren oder genaueren Auswertung auf geeignete Weise miteinander verknüpft werden. Die kapazitive Messung hat den Vorteil, dass sie ein Signal mit einem guten Signal/Rauschverhältnis liefert. Das Signal ist aber proportional zur Masse pro Längeneinheit und entspricht somit nicht dem visuellen Eindruck vom Garn; dies wirkt sich gerade bei Effektgarn, das im Bereich eines Effektes 92 oft eine andere Garndichte aufweist als im Bereich eines Stegs 91, nachteilig aus. Die optische Messung hat den Vorteil, den visuellen Eindruck vom Garn besser wiederzugeben, weil sie im Wesentlichen den sichtbaren Garndurchmesser misst; deshalb eignet sie sich besser für Gewebesimulationen. Dafür weist das optische Messsignal ein höheres Rauschen auf als das kapazitive Messsignal. Durch geeignete Verknüpfung der beiden Ausgangssignale ist es möglich, von den Vorteilen beider Messarten zu profitieren und ihre jeweiligen Nachteile zu eliminieren oder zumindest abzuschwächen.

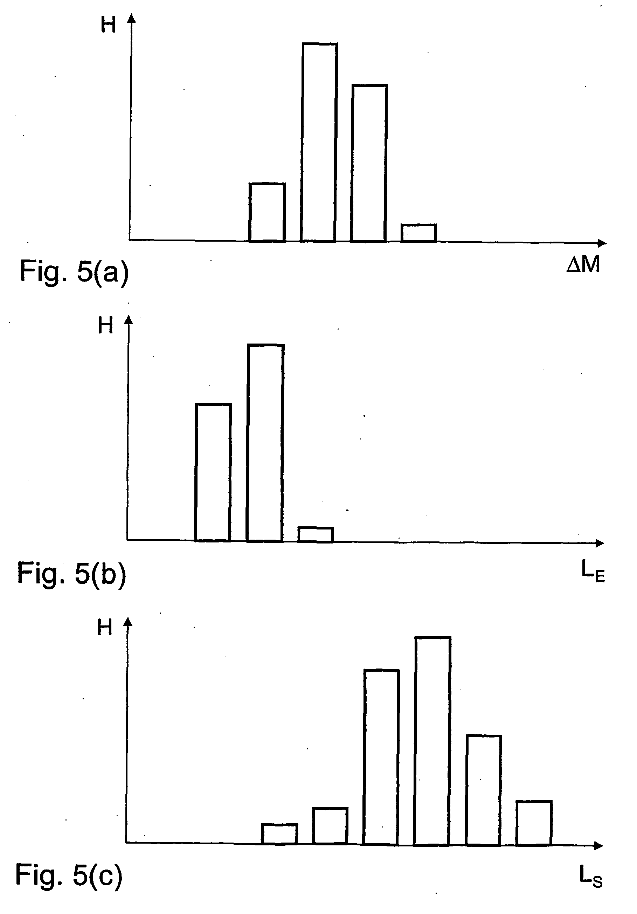

Resultate der Auswertung können einerseits Grössen sein wie z. B. Minima, Maxima, arithmetische Mittel und/oder Standardabweichungen der oben definierten Effektgarnparameter. Die Anzahl Effekte 92 pro Garnlänge kann eine weitere interessierende Grösse sein. Diese Grössen können als alphanumerische Zeichen ausgegeben werden. Andererseits können die Resultate der Auswertung in geeigneter Weise auf der Ausgabeeinheit 4 grafisch dargestellt und ausgegeben werden. Bevorzugte Darstellungsarten sind in den Figuren 5-11 gezeigt.

Eine mögliche Darstellungsart ist ein Histogramm, d. h. die grafische Darstellung der Klassenhäufigkeiten der klassierten Garnparameter. Drei Beispiele in Form von Säulendiagrammen sind in **Figur 5** angegeben. Die Ordinate ist jeweils die Häufigkeit H. Als Abszisse wurde in **Figur 5(a)** die Massenzunahme ΔM, in **Figur 5(b)** die Effektlänge L_{E} und in **Figur 5(c)** die Steglänge L_{S} verwendet. Die Abszissen können eine lineare, logarithmische oder andere Einteilung aufweisen. Einteilung und/oder Skalierung der Achsen können automatisch berechnet oder mittels Eingabe durch eine Bedienungsperson gewählt werden. Dasselbe gilt für die Wahl der Klassierung, d. h. für die Breite der Klassen. Es müssen nicht notwendigerweise alle Klassen dieselbe Breite aufweisen.

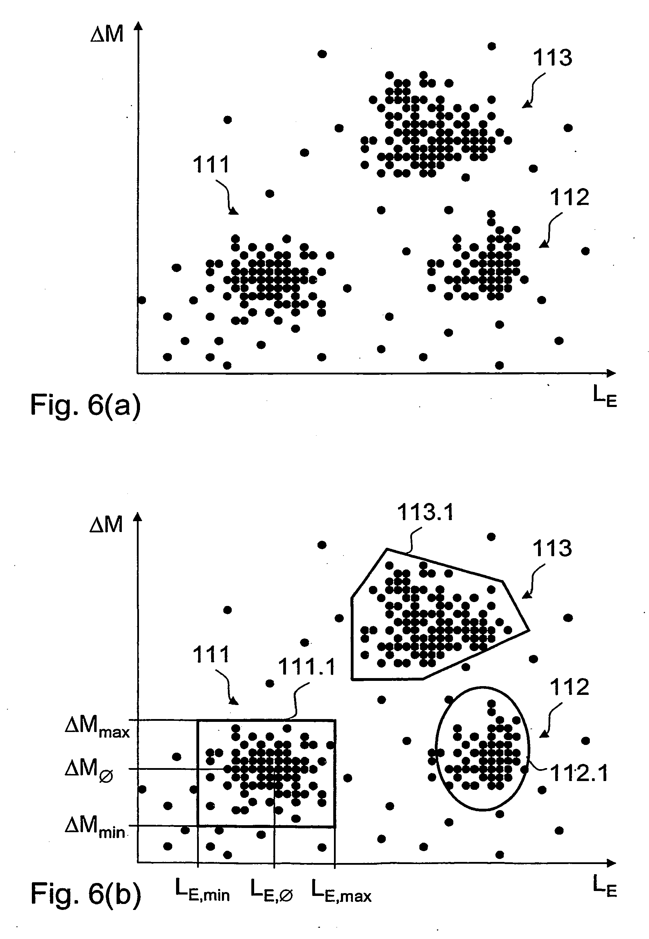

Die Darstellungsart von **Figur 6** ist ein so genannter Scatterplot (Punktwolke). Darin ist für alle Effekte 92 eines Effektgarns 9 oder eines Garnabschnitts die Massenzunahme ΔM gegenüber der Effektlänge L_{E} aufgetragen. Jeder Effekt 92 wird als Punkt an der richtigen Stelle eingetragen. Diese Darstellung erleichtert die Einteilung verschiedener Effekte 92 in verschiedene Klassen. So ist im Beispiel von **Figur 6(a)** sofort ersichtlich, dass das untersuchte Effektgarn 9 drei Klassen 111-113 oder Populationen von Effekten 92 aufweist:
- eine erste Klasse 111 mit kurzen, niedrigen Effekten,
- eine zweite Klasse 112 mit langen, niedrigen Effekten, und
- eine dritte Klasse 113 mit langen, hohen Effekten.

Anhand des Scatterplots von Fig. 6(a) kann eine Ausreisseranalyse vorgenommen werden. Zu diesem Zweck kann ein Werkzeug vorgesehen sein, um Effektpopulationen 111-113 zu definieren und voneinander sowie von Ausreissern abzugrenzen. Ein solches Werkzeug kann es z. B. ermöglichen, auf einem Bildschirm mit einer Maus Teilflächen 111.1-113.1 des Scatterplots zu definieren, wie in **Figur 6(b)** dargestellt. Jeder Teilfläche 111.1-113.1 wird eine Effektpopulation 111-113 zugewiesen. Die Teilflächen können z. B. als Rechteck 111.1, als Kreis 112.1 oder als Polygonzug 113.1 geformt sein. Ausserhalb der Teilflächen 111.1-113.1 liegende Punkte gelten als Ausreisser und können bei weiteren Auswertungen und/oder Darstellungen als solche gekennzeichnet werden oder unberücksichtigt bleiben. Wird keine Teilfläche definiert, so gelten alle Punkte des Scatterplots als Effekte und werden als solche behandelt.

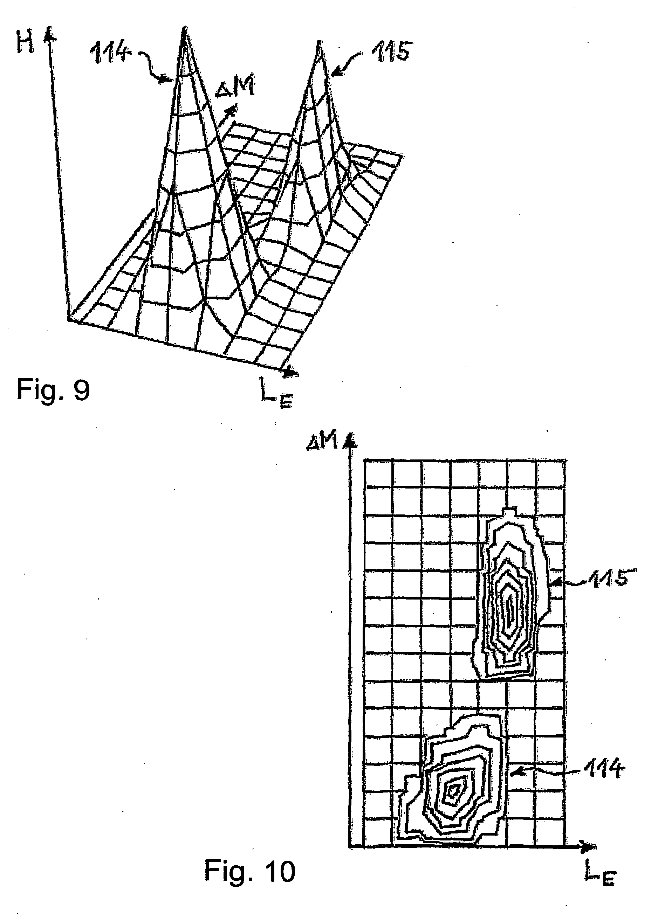

Andere Scatterplots sind möglich, bspw. Effektgesamtmasse M_{E} versus Effektlänge L_{E}, Effektgesamtmasse M_{E} versus Massenzunahme ΔM etc. Der Scatterplot kann farbig ausgegeben werden, wobei verschiedene Farben verschiedene Messungen, verschiedene Punktdichten und/oder Populationen bzw. Ausreisser (vgl. Fig. 10) bedeuten können. Analog ist eine dreidimensionale Darstellung möglich, in der zwei Koordinaten denjenigen von Fig. 6 entsprechen und die dritte Koordinate der Punktdichte entspricht; vgl. Fig. 9.

Der Scatterplot kann für eine einzelne Effektgarnprobe oder für mehrere Effektgarnproben dargestellt werden. Im letzteren Fall können verschiedene Farben für die verschiedenen Effektgarnproben verwendet werden, um mögliche Unterschiede zwischen den Proben aufzuzeigen. Bei mehreren Effektgarnproben kann zusätzlich zu den Resultaten der einzelnen Effektgarnproben das Resultat der Gesamtheit aller Effektgarnproben angezeigt werden, vorzugsweise in einer eigens zugeordneten Farbe.

Auf dem Scatterplot können nebst den Istwerten auch Sollwerte oder Sollbereiche für eine oder mehrere Klassen von Effekten dargestellt werden. Soll- und Istwerte können auch in anderen Darstellungsarten oder rein numerisch verglichen werden. Derartige Soll-Ist-Vergleiche ermöglichen z. B. eine Kontrolle über die Güte einer Kopie (Istwert) eines vorgegebenen Effektgarns (Sollwert).

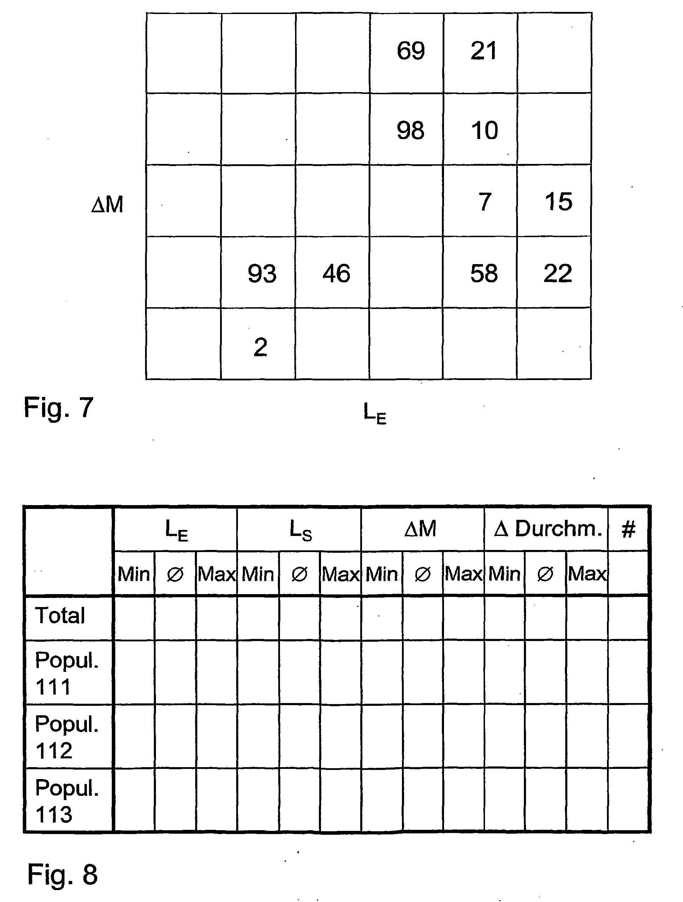

Statt eines Scatterplots können die Resultate in Form einer Tabelle oder Klassiermatrix - wie in **Figur 7** dargestellt - ausgegeben werden. Die Tabellenachsen entsprechen den Achsen des Scatterplots von Fig. 6. In die Tabellenfelder werden die Anzahlen von entsprechenden Effekten 92 eingetragen. Alternativ zur absoluten Anzahl von Effekten 92 kann auch deren relativer Anteil, z. B. in Prozent oder Promille, angegeben werden. Jedes Tabellenfeld stellt somit eine Klasse von Effekten 92 dar, analog zu den anlässlich von Fig. 6 beschriebenen Klassen 111-113. Die gewählte Grösse der Tabellenfelder richtet sich nach der angestrebten Klassierung. Bei der Wahl der Grösse der Tabellenfelder sollte ferner berücksichtigt werden, dass die Auflösung genügend fein ist, aber die Tabelle noch übersichtlich bleibt. Im Beispiel von Figur 7 wurden relativ kleine Tabellenfelder gewählt, die eine feinere Klassierung erlauben als in die drei Klassen 111-113, die anlässlich von Figur 6 identifiziert wurden. Zwecks besserer Visualisierung können die Tabellenfelder mit Farben, Mustern oder Graustufen gefüllt werden, die wiederum verschiedenen Anzahlen von Effekten 92 zugeordnet sind.

Sowohl beim Scatterplot (Fig. 6) als auch bei der Klassiermatrix (Fig. 7) können die Achsen, unabhängig voneinander, eine lineare, logarithmische oder andere Einteilung aufweisen. Einteilung und/oder Skalierung der Achsen können automatisch berechnet oder mittels Eingabe durch eine Bedienungsperson gewählt werden. Dasselbe gilt für Breite und Höhe der Tabellenfelder in der Klassiermatrix von Figur 7, d. h. für die Wahl der Klassierung. **Figur 8** zeigt eine bevorzugte Ausgabemöglichkeit für die ermittelten Effektgarnparameter. Es handelt sich um eine Tabelle, die in fünf Hauptkolonnen für die fünf Parameter Effektlänge L_{E}, Steglänge L_{S}, Massenzunahme ΔM, Durchmesserzunahme und Anzahl # der Effekte unterteilt ist. Die ersten vier Hauptkolonnen sind ihrerseits in jeweils drei Unterkolonnen für das Minimum Min, den Mittelwert Ø und das Maximum Max des entsprechenden Parameters unterteilt. Die Zeilen der Tabelle können die Werte für das gesamte Garn oder den gesamten untersuchten Garnabschnitt, sowie für die einzelnen Effektpopulationen 111-113 enthalten. Die Minima L_{E,min}, ΔMₘᵢₙ, die Mittelwerte L_{E,Ø}, ΔM_{Ø} und die Maxima L_{E,max}, ΔMₘₐₓ sind für die Population 111 in Fig. 6(b) angedeutet. Selbstverständlich kann die Tabelle auch anders gestaltet sein. Jedenfalls führt eine solche tabellarische Darstellung der ermittelten Effektgarnparameter zu einer Datenreduktion. Anhand der Tabelle lassen sich Besonderheiten des betreffenden Effektgarns schnell erfassen, und verschiedene Effektgarne lassen sich leicht vergleichen.

Eine weitere Darstellungsart für Resultate der Auswertung von Effektgarnparametern zeigt **Figur 9** Es handelt sich um eine Fläche in drei Dimensionen (3D). Zwei der drei Dimensionen, die beiden horizontalen Achsen, entsprechen der Effektlänge L_{E} bzw. der Massenzunahme ΔM, wie im Scatterplot von Fig. 6. Die dritte, vertikale Dimension gibt die entsprechende Häufigkeit H der gemessenen Werte an, d. h. die Punktdichte im Scatterplot von Fig. 6 oder die Anzahlen von Fig. 7. Die so entstandene 3D-Fläche vermittelt den Eindruck eines Gebirges, das eine rasche und einprägsame visuelle Erfassung der Eigenarten des jeweiligen Effektgarns 9 erlaubt. Besonders ein synoptischer Vergleich zweier solcher "Gebirge" zeigt sehr schnell, ob die betreffenden Effektgarne ähnliche oder unterschiedliche Charakteristika aufweisen, und im letzteren Fall, wo die Hauptunterschiede liegen. Zu bemerken ist, dass sich das Beispiel von Fig. 9 auf ein anderes Effektgarn bezieht als das Beispiel von Fig. 6. Während das Effektgarn von Fig. 6 drei Klassen 111-113 von Effekten 92 aufweist, hat das Effektgarn von Fig. 9 nur deren zwei 114, 115.

Die dreidimensionale Darstellungsart von Fig. 9 lässt sich auch auf zwei Dimensionen reduzieren. **Figur 10** zeigt ein solches Diagramm, das durch eine Projektion des "Gebirges" von Fig. 9 in die durch die beiden horizontalen Achsen L_{E}, M aufgespannte Ebene entsteht. So ergibt sich eine "Landkarte", auf der die "Berge" 114, 115 von Fig. 9 mittels "Höhenlinien", d. h. Linien gleicher Häufigkeit H, dargestellt sind. Statt "Höhenlinien" können Farben oder Schraffuren zur Sichtbarmachung der verschiedenen Häufigkeiten H verwendet werden.

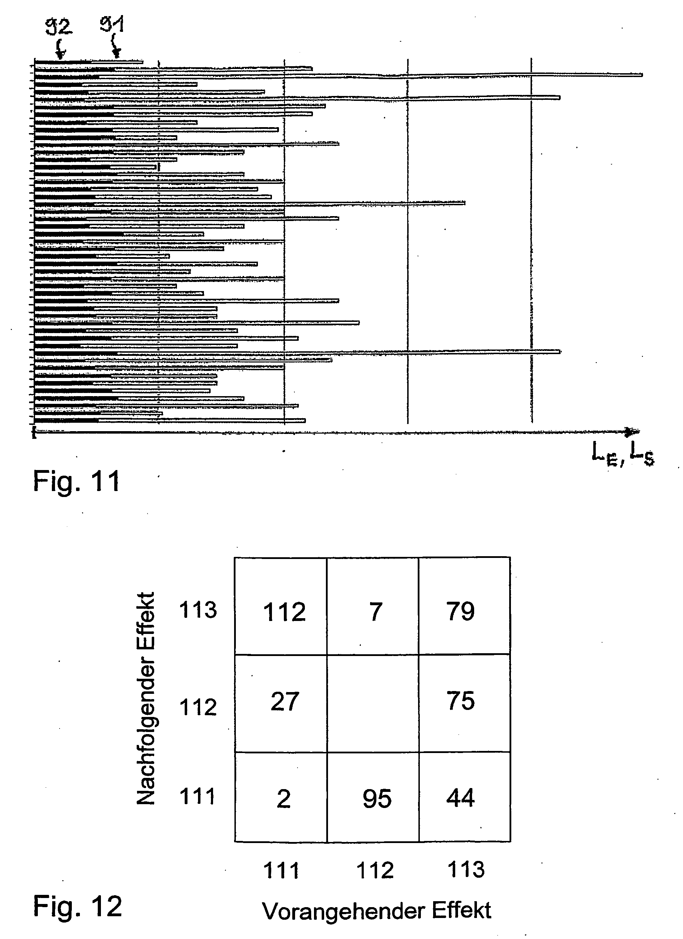

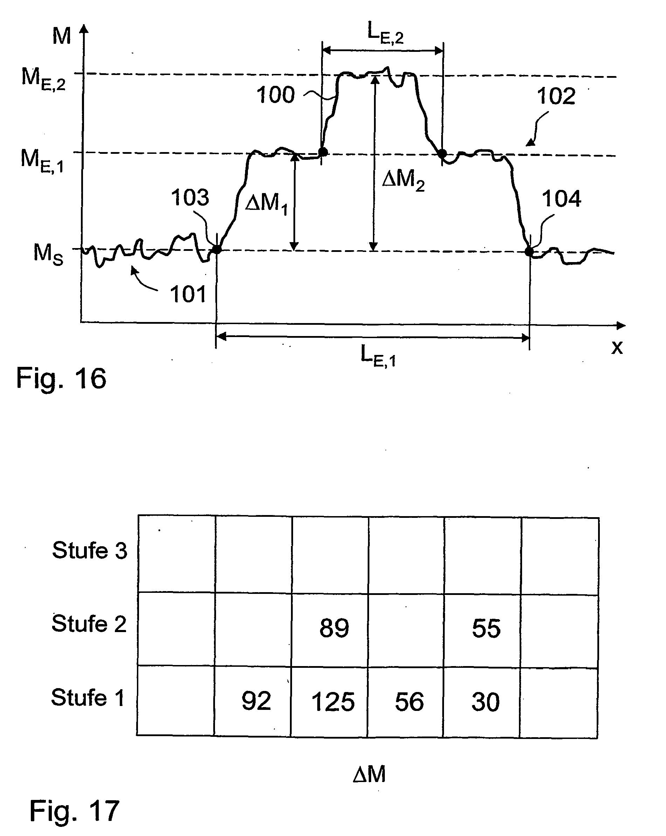

Die Darstellungsarten der Figuren 5-10 lassen Information über die Abfolge der einzelnen Effekte unberücksichtigt. Diese Information ist in der Messreihe, wie sie in Figur 2 dargestellt ist, vollständig vorhanden. Es ist möglich, daraus die entsprechenden Garnparameter wie Massenzunahme, Effektlänge und Steglänge als Messgrössen (Zahlenwert x Einheit) zu ermitteln und diese Messgrössen in der Reihenfolge ihres Auftretens nacheinander aufzulisten. Eine solche alphanumerische Auflistung der reduzierten Messdaten kann für bestimmte Fälle nützlich sein, ist jedoch wenig übersichtlich. In der Darstellungsart von **Figur 11** ist die Information über die Abfolge der einzelnen Effekte vollständig erhalten, wobei die Grafik gegenüber einer alphanumerischen Wertetabelle den Vorteil einer besseren Übersichtlichkeit und visuellen Erfassbarkeit bietet. Für jeden Effekt 92 und einen benachbarten Steg 91 ist jeweils ein horizontaler Balken eingezeichnet. Der Balken setzt sich aus zwei Teilen zusammen. Die Länge eines ersten, linken Teils gibt die jeweilige Effektlänge L_{E} an, die Länge eines zweiten, rechten Teils die jeweilige Steglänge L_{S}. Der nächste, darunter liegende Balken charakterisiert den nachfolgenden Effekt, usw. Selbstverständlich könnten die horizontalen Balken von Fig. 11 durch vertikale Säulen ersetzt werden. Statt Längen L_{E}, L_{S} können andere Messgrössen als Balken aufgetragen werden, z. B. eine Effektmasse und die dazugehörige Stegmasse, was besonders bei strukturiertem Garn mit unterschiedlichen Stegmassen vorteilhaft sein kann. Auch ist es möglich, dass ein Balken oder eine Säule mehr als zwei Effektparameter angibt, z. B. bei mehrstufigen Effekten (siehe Fig. 16) die erste Effektlänge L_{E,1}, die zweite Effektlänge L_{E,2} und die dazugehörige Steglänge L_{S}.

**Figur 12** zeigt eine Darstellungsart, welche zumindest einen Teil der Information über die Abfolge der einzelnen Effekte übersichtlich wiedergibt. Es handelt sich um eine Klassiermatrix, die eine Klassierung der Effekte voraussetzt, wie sie etwa in Figur 6 mit den Klassen 111-113 vorgenommen wurde. Es wird jeweils ein Paar von zwei benachbarten Effekten 92, 92' betrachtet, von denen ein erster Effekt 92 "vorangehender Effekt" und ein zweiter Effekt 92' "nachfolgender Effekt" genannt wird. Für jedes Paar 92, 92' erfolgt ein entsprechender Eintrag in die Klassiermatrix von Figur 12, deren horizontale Achse die vorangehenden Effekte 92 und deren vertikale Achse die nachfolgenden Effekte 92' angibt. Aus dem fiktiven Beispiel von Figur 12 kann man herauslesen, dass praktisch nie zwei Effekte der ersten Klasse 111 (vgl. Fig. 6) und nie zwei Effekte der zweiten Klasse 112 aufeinander folgen, dass hingegen oft ein Effekt der ersten Klasse 111 auf einen Effekt der zweiten Klasse 112 folgt und sehr oft ein Effekt der dritten Klasse 113 auf einen Effekt der ersten Klasse 111 folgt.

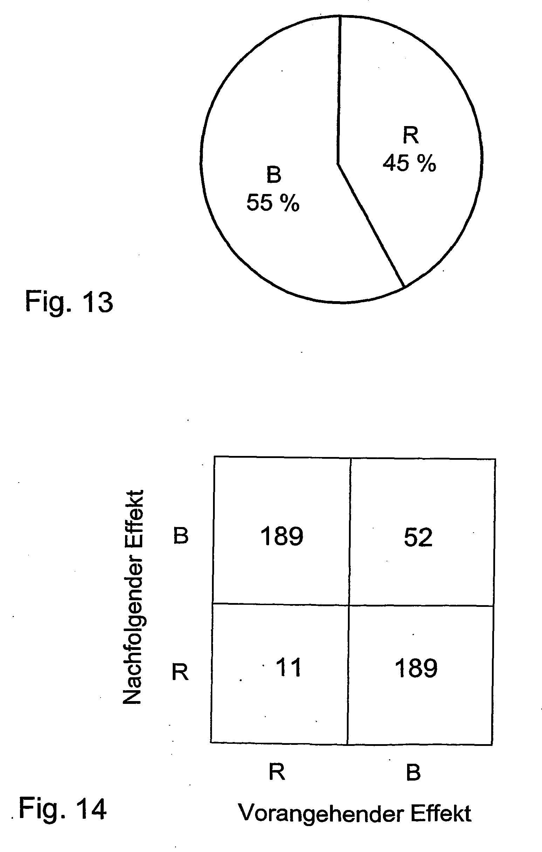

Verschiedene Effekte eines Effektgarns 9 können verschiedene Farben aufweisen. Es kann daher wünschenswert sein, Information über die Farbe von Effektgarn 9 zu gewinnen. Dazu geeignete Abtasteinheiten 2. und Auswerteeinheiten 3 (siehe Fig. 1) sind aus dem oben zitierten Stand der Technik bekannt. Eine mögliche Darstellungsart für die gewonnene Farbinformation ist in **Figur 13** gezeigt. Dabei handelt es sich um ein Kreisdiagramm, das die gemessenen Anteile der verschiedenfarbigen Effekte angibt. Im Beispiel von Figur 13 enthielt das Effektgarn rote (R) und blaue (B) Effekte, die mit einer Häufigkeit von 45 % bzw. 55 % auftraten. Eine Erweiterung auf mehr als zwei Farben ist selbstverständlich möglich. Falls die Ausgabeeinheit 4 (siehe Fig. 1) eine farbige Ausgabe erlaubt, können die einzelnen Kreissegmente in der entsprechenden Farbe ausgegeben werden. Statt eines Kreisdiagramms ist auch ein Kuchendiagramm möglich.

Das Kreisdiagramm von Figur 13 beinhaltet keine Information über die Abfolge der Farbeffekte. Diese Information ist in der Darstellungsart von **Figur 14** zumindest teilweise vorhanden. Analog zu Figur 12 werden jeweils zwei aufeinander folgende Effekte betrachtet und die Häufigkeiten ihrer Farben R, B in die Tabelle eingetragen, wobei die horizontale Tabellenachse die Farbe R, B des vorangehenden Effekts und die vertikale Achse die Farbe R, B des nachfolgenden Effekts angibt. Aus der Tabelle von Figur 14 lässt sich herauslesen, dass in diesem fiktiven Beispiel Farbwechsel häufig vorkommen, während dagegen zwei gleichfarbige benachbarte Effekte eher selten sind.

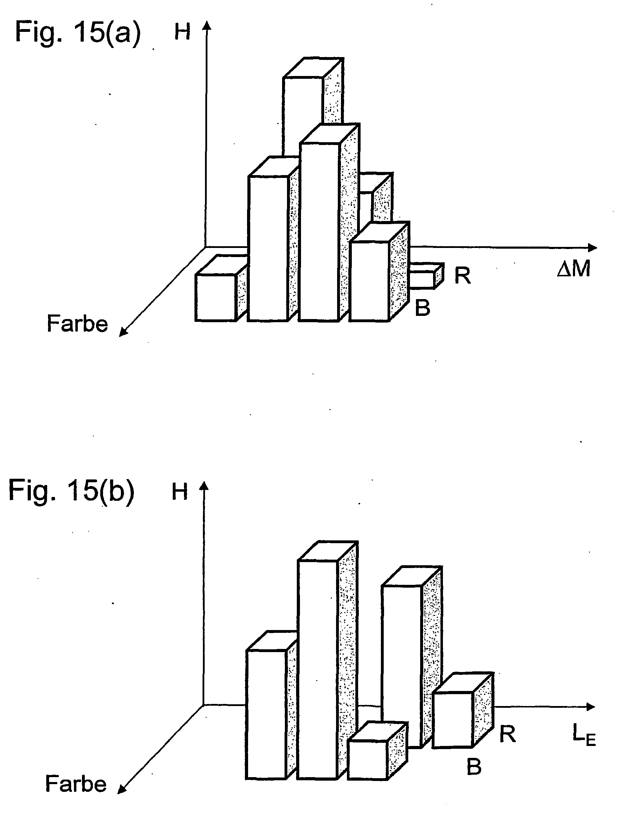

Die dreidimensionalen Säulendiagramme von **Figur 15** zeigen anhand von fiktiven Beispielen, wie sich die Farbinformation mit der geometrischen Information in einer einzigen Darstellungsart verknüpfen lässt. Dabei liegt die geometrische Information in der Zeichenebene und entspricht derjenigen von Fig. 5(a) bzw. 5(b). Im Diagramm von **Figur 15(a)** ist die Häufigkeit von Klassen der Massenzunahme ΔM, im Diagramm von **Figur 15(b)** die Häufigkeit von Klassen der Effektlänge L_{E} aufgetragen. Die dritte Dimension wird für die Farbinformation R, B gebraucht. Aus dem Diagramm von Figur 15(a) lässt sich herauslesen, dass die roten Effekte R eher kleinere Massenzunahmen ΔM aufweisen als die blauen Effekte B. Das Diagramm von Figur 15(b) sagt aus, dass die roten Effekte R eher länger sind als die blauen Effekte B.

Die oben diskutierten Figuren 5-15 geben nur einige Beispiele für die Darstellung der Effektgarnparameter Massenzunahme ΔM (bzw. Durchmesserzunahme), Effektlänge L_{E}, Steglänge L_{S} und Farbe an. Selbstverständlich können weitere Beziehungen zwischen diesen und weiteren Effektgarnparametern in ähnlicher Weise graphisch zwei- oder dreidimensional dargestellt werden.

Während bisher einstufige Effekte behandelt wurden, werden nachfolgend mehrstufige Effekte behandelt. Ein Beispiel einer Reihe von Messwerten an einem zweistufigen Effektgarn ist in **Figur 16** angegeben, in derselben Darstellung wie Fig. 4. Hier kann unterschieden werden zwischen einer ersten Effektstufe mit einer ersten Effektgesamtmasse M_{E,1} pro Längeneinheit, einer ersten Massenzunahme AM₁ und einer ersten Effektlänge L_{E,1}, und einer zweiten Effektstufe mit einer zweiten Effektgesamtmasse M_{E,2} pro Längeneinheit, einer zweiten Massenzunahme ΔM₂ und einer zweiten Effektlänge L_{E,2}. Die genannten Parameter können analog zur oben für einstufiges Effektgarn beschriebenen Weise ermittelt werden.

Eine mögliche Darstellungsart für die Parameter von mehrstufigem Effektgarn zeigt **Figur 17****.** Es handelt sich um eine Tabelle, deren horizontale Achse der klassierten Massenzunahme ΔM entspricht; vgl. Fig. 5(a). Die Zeilen der Tabelle stehen für die verschiedenen Stufen des Effektgarns. In die Tabellenfelder werden die entsprechenden Häufigkeiten eingetragen. Im fiktiven Beispiel von Figur 17 ist das Effektgarn zweistufig, wobei die zweite Stufe in zwei Varianten vorkommt: mit relative kleiner Massenzunahme ΔM₂ einerseits und mit relativ grosser Massenzunahme ΔM₂ andererseits. Eine analoge Darstellungsart ist auch für die Effektlänge L_{E,1}, L_{E,2},... möglich.

Ein weiterer Parameter von Effektgarn ist die so genannte Rapportlänge. Dies ist die Länge der kürzesten Sequenz von Effekten, die sich im Effektgarn periodisch wiederholt. Innerhalb dieser Sequenz gibt es keinerlei Periodizität, d. h. mindestens ein Effektparameter wie z. B. die Steglänge L_{S} ist zufällig oder pseudozufällig verteilt. Die Rapportlänge kann z. B. mittels Korrelationsrechnung aus den Messwerten, wie sie für einen kurzen Garnabschnitt in Figur 2. dargestellt sind, gewonnen werden. Eine solche Korrelationsrechnung mit allen Messwerten kann rechnerisch aufwändig sein. Um den Rechenaufwand zu vermindern, können die Messdaten vorgängig reduziert werden, indem z. B. die entsprechenden Garnparameter wie Massenzunahme, Effektlänge und Steglänge ermittelt werden und die Korrelationsrechnung auf diesen reduzierten Daten basiert. Auf analoge Weise kann auf Information über das Vorhandensein von - meist unerwünschten - Unterrapporten und deren Längen gewonnen werden. Die Rapportlänge und/oder die Unterrapportlänge werden vorzugsweise in alphanumerischer oder grafischer Form ausgegeben.

Nützliche Information über das Effektgarn kann auch ein Spektrogramm des Messsignals M(x) von Figur 2 liefern. Zur Ermittlung des Spektrogramms wird das Messsignal M(x) vorzugsweise einer Fouriertransformation unterzogen. Ein fiktives Beispiel eines Spektrogramms |F {M}|, genauer des Realteils der Fouriertransformierten, ist in **Figur 18** gezeigt, wobei usanzgemäss als Abszisse eine Periodenlänge L, vorzugsweise in logarithmischem Massstab, gewählt wurde. Üblicherweise wird das Spektrogramm |F {M}| eine relativ breite Verteilung 131 der Ortsfrequenzen bzw. der Periodenlänge L zeigen. Aus der Lage des Maximums 132 kann auf einen mittleren Abstand der Effekte geschlossen werden. Eine ausgeprägte Spitze im Spektrogramm |F {M}| würde auf eine - meist unerwünschte - Periodizität im Effektgarn hinweisen. Die Periodizität kann sich einerseits auf die einzelnen Effekte beziehen. Bei Effektgarn mit konstantem Effektabstand L_{E} + L_{S}, innerhalb dessen die Effektlänge L_{E} und die Steglänge L_{S} variieren, erscheint das Maximum 132 als ausgeprägte Spitze. Um dies festzustellen, sollte eine Garnlänge von mindestens zehn, vorzugsweise hundert und mehr Effektabständen ausgemessen werden. Andererseits kann sich die Periodizität auf die Rapporte beziehen. Bei einer genügend langen Messreihe - mindestens zehn, vorzugsweise aber hundert und mehr Rapportlängen - lässt sich aus der Lage einer entsprechenden Spitze 133 im langwelligen Bereich des Spektrogramms |F {M}| auch die Rapportlänge herauslesen.

Eine weitere grafische Darstellungsart für die Effekte ist eine geglättete oder idealisierte Darstellung der Messwerte, wie dies in **Figur 19** gezeigt ist. In Figur 19 sind einerseits, gestrichelt, die Messwerte von Figur 4 aufgezeichnet. Andererseits ist in ausgezogenen Linien ein idealisierter Verlauf der Messkurve angegeben. Wie der Fachmann weiss, gibt es viele Möglichkeiten, aus einer realen Messkurve eine idealisierte Kurve in der Art von Figur 19 zu erhalten. Im Anwendungsbeispiel von Figur 19 wurden Stege 91, 91' durch waagrechte Geraden angenähert, die alle auf der Höhe der zuvor ermittelten Stegmasse M_{S} liegen. Ein Effekt 92 wird jeweils als Trapez mit Flanken 93, 94 und einem waagrechten Dach 95 idealisiert. Dabei muss das Trapez nicht notwendigerweise symmetrisch sein, d. h. die Flanken 93, 95 können auch betragsmässig unterschiedliche Steigungen haben. Die Annäherung des Effektes 92 mit einem Trapez kann nach verschiedenen, dem Fachmann bekannten Verfahren und Kriterien erfolgen. Die Flanken 93, 94 können etwa Geraden sein, deren Lagen mittels der kleinsten Summe der Quadrate oder auf andere Weise ermittelt wurden. Die Höhe des Trapezes, d. h. die Lage des Daches 95, kann gemäss dem Kriterium ermittelt werden, dass die Fläche des Trapezes gleich der Fläche unter der realen Messkurve ist. Die Effektlänge L_{E} kann bspw. als Basislänge des Trapezes definiert werden, d. h. als die Distanz zwischen einem Effektbeginn 103* und einem Effektende 104*, wobei der Effektbeginn 103* und das Effektende 104* die Schnittpunkte der die Stegmasse M_{S} darstellenden Waagrechten und der linken Flanke 93 bzw. der rechten Flanke 94 sind. Alternativ kann die Effektlänge L_{E} als Breite des Trapezes auf halber Höhe definiert werden, was der Hälfte der Summe von Basislänge und Dachlänge gleich ist. Die Massenzunahme ΔM kann als die Höhe des Trapezes, d. h. als Abstand zwischen der Basis und dem Dach, definiert werden. Unter Umständen kann das Trapez zum Spezialfall eines Dreiecks (Trapez mit Dachlänge gleich null) oder eines Rechtecks (Trapez mit lauter rechten Winkeln) degenerieren. Andere Formen für die idealisierte Messkurve sind ebenfalls möglich. Derartige idealisierte Kurvenverläufe erlauben eine bessere visuelle Erfassung der Charakteristika des Effektgarns. Parameter der idealisierten Kurve, wie z. B. die Höhe, die Basislänge, die Dachlänge, die Effektlänge L_{E} oder die Flankensteigungen des Trapezes, können als charakteristische Grössen bspw. in einer Tabelle ausgegeben und für weitere Auswertungen verwendet werden. Die Ausgabe solcher Grössen führt zu einer Reduktion der Daten.

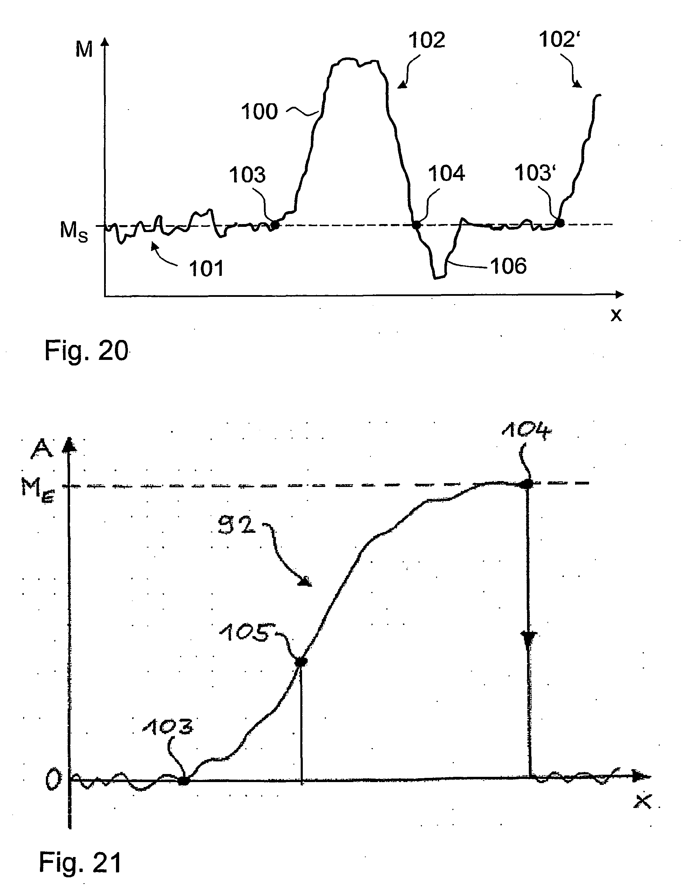

Ein unvollkommener Herstellungsprozess für das Effektgarn kann dazu führen, dass unmittelbar neben einem Effekt 102 eine Dünnstelle 106 liegt, wie in **Figur 20** angedeutet. Gemäss der vorliegenden Erfindung können solche unerwünschten Dünnstellen 106 erfasst werden. Ihre Anzahl und/oder ihr mengenmässiger Anteil an den Effekten 102 kann als Resultat ausgegeben werden. Ein Dünnstellenanteil von 50 % bedeutet, dass neben der Hälfte aller Effekte 102 eine Dünnstelle 106 beobachtet wurde, was auf einen mangelhaften Herstellungsprozess hinweist

Spinnereien, die Effektgarn herstellen, haben das Bedürfnis, zwischen den folgenden beiden Phänomenen zu unterscheiden:
- einerseits der Grundgarnherstellung, die Imperfektionen, Unregelmässigkeiten und Fehler wie Dick- oder Dünnstellen ins Garn einbringen kann, und
- andererseits der Effektherstellung, die erwünschte Effekte, z. B. in Form von Verdickungen, auf das Grundgarn aufbringt.
Diese beiden Phänomene sind mit herkömmlichen Garnprüfverfahren und -geräten manchmal nicht oder schwer zu unterscheiden. Die beispielhafte Kurve von Fig. 4 wurde aus didaktischen Gründen so gewählt, dass daraus recht klar ersichtlich ist, was ein Effekt 102 und was ein Steg 101 ist. In der Praxis können aber die unerwünschten Dickenschwankungen auf dem Steg 101 so gross sein, dass sie den Schwellenwert M_{T} überschreiten und folglich bei der Auswertung irrtümlicherweise für einen Effekt gehalten werden. So werden die Resultate der Auswertung verfälscht. Dies kann zur Folge haben, dass im Herstellungsprozess falsche Massnahmen getroffen werden. Wenn z. B. lange Dickstellen oder Garnmassenschwankungen für kleine Effekte gehalten werden, wird der Teilprozess zur Effektherstellung so geändert, dass grössere Effekte erzeugt werden. Diese Massnahme verändert unnötigerweise die Effektstruktur, ohne die eigentliche Fehlerursache - vielleicht einen Defekt in einer Strecke - zu beheben.

Eine bevorzugte Ausführungsform schafft hier Abhilfe, indem sie bei der Definition eines Effektes ansetzt. Das anlässlich von Fig. 4 beschriebene Verfahren kann nachteilig sein, weil es mit der Vorgabe des Schwellenwertes M_{T} nur die eindimensionale Massenzunahme berücksichtigt. Wie beschrieben, kann man zwar versuchen, diesen Nachteil durch die Forderung abzuschwächen, das Überschreiten müsse über eine gewisse Länge andauern. Aber auch dies führt nicht zu einer optimalen Erkennung der Effekte. Als vorteilhafter hat es sich erwiesen, eine Fläche unter der Messkurve - oder, was hier als äquivalent zur Fläche betrachtet wird, ein bestimmtes Integral der Messkurve - zu betrachten. Eine solche Fläche kann mit einem der bekannten numerischen Integrationsverfahren, etwa mit dem Rechteck- oder dem Trapezverfahren, zumindest näherungsweise berechnet werden. **Figur 21** zeigt schematisch einen typischen Verlauf der auf diese Weise berechneten Fläche A(x) als Funktion der Längskoordinate x, wobei als Basis für die Flächenbestimmung die zuvor bestimmte Stegmasse M_{S} (siehe Fig. 4) dient. Im Bereich eines Steges 101 schwanken die Werte von A(x) um den Wert null herum. Etwaige Dickstellen, auch wenn sie eine grosse Massenzunahme haben, verursachen keine signifikanten Flächenänderungen, weil sie sich nur über jeweils ein kurzes Längenintervall erstrecken. Ein richtiger Effekt 92 unterscheidet sich von einer Dickstelle, indem er einen signifikanten Anstieg der Kurve A(x) verursacht, der bei einem Effektbeginn 103 einsetzt. Ein lokales Maximum 105 des Effektes 92, das auch als Position des Effektes 92 bezeichnet werden kann, befindet sich beim Wendepunkt der Kurve A(x). Nach einem Effektende 104 schwankt die Kurve A(x) wieder um einen konstanten Wert herum, welcher die Effektgesamtmasse M_{E} angibt. Wird ein solches Effektende 104 festgestellt, kann der weitere Kurvenverlauf mittels Subtraktion des Wertes M_{E} auf den Wert null zurückgesetzt werden. Es folgt ein weiterer Steg, usw. Auf diese Weise können alle Effekte und ihre Positionen zuverlässig und stabil erkannt und von Dickstellen unterschieden werden.

Vorzugsweise wird die Feststellung eines Effektes vom gleichzeitigen Erfüllen mehrer Kriterien abhängig gemacht, z. B. der folgenden drei Kriterien:
(i) Überschreiten eines vorgegebenen Schwellenwertes für die Fläche A(x),
(ii) Überschreiten eines vorgegebenen Schwellenwertes für die Effektlänge L_{E}, und
(iii) Überschreiten eines vorgegebenen Schwellenwertes für die Massenzunahme ΔM.
Nur wenn diese Kriterien gleichzeitig erfüllt sind, kann zuverlässig davon ausgegangen werden, dass ein richtiger Effekt und nicht eine Garnimperfektion vorliegt.

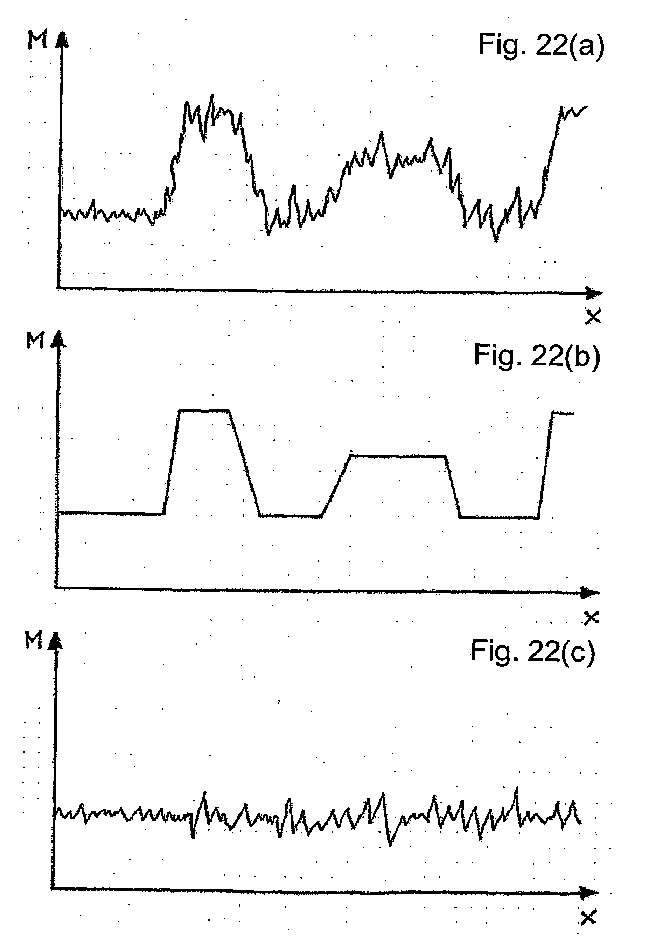

Die Unterscheidung zwischen Grundgarn und Effekten wird durch eine weitere bevorzugte Ausführungsform des erfindungsgemässen Verfahrens noch mehr erleichtert. Gemäss dieser Ausführungsform wird der idealisierte Kurvenverlauf gemäss Fig. 19 von der realen Messkurve subtrahiert. Dies ist schematisch in **Figur 22** dargestellt. Darin zeigt **Figur 22(a)** die Kurve der ursprünglichen Messwerte, **Figur 22(b)** die idealisierte Kurve (vgl. Fig. 19) und **Figur 22(c)** die Kurve, die entsteht, wenn man die idealisierte Kurve von der ursprünglichen Messkurve subtrahiert. Die Kurve von Fig. 22(b) zeigt nur die (idealisierten) Effekte, die Kurve von Fig. 22(c) nur das Grundgarn ohne Effekte. Schon diese Darstellungen können zu einem tieferen Verständnis der Struktur des untersuchten Effektgarns beitragen. Die so gewonnenen Daten können aber noch weiter ausgewertet werden, wie nachfolgend diskutiert wird.

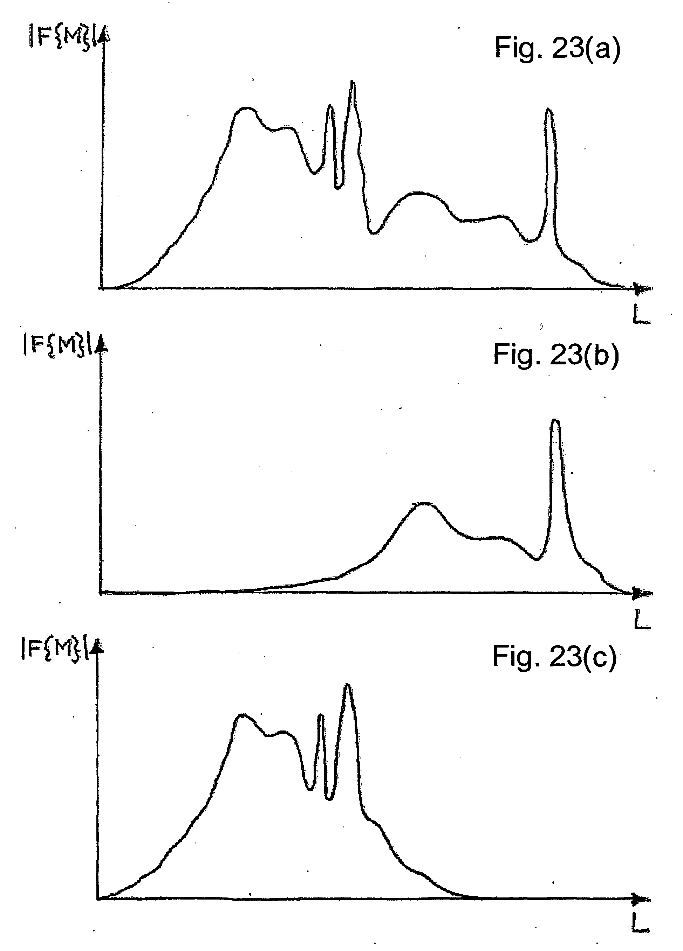

**Figur 23** zeigt schematisch Resultate einer Auswertung der Daten von Fig. 22 einem Spektrogramm, d. h. in einer Darstellung, die derjenigen von Fig. 18 entspricht. **Figur 23(a)** zeigt das Spektrogramm der ursprünglichen Messwerte, d. h. der Kurve von Fig. 21 (a). Wie oben bereits angedeutet, ist es nur anhand dieses Spektrogramms schwierig oder gar unmöglich, zwischen Effekten und Grundgarn zu unterscheiden. Die Darstellungen der Figuren 23(b) und (c) schaffen Abhilfe. **Figur 23(b)** zeigt das Spektrogramm der Effekte allein, d. h. der Kurve von Fig. 22(b). Diese effektbezogenen Daten erlauben es, gezielt in der Effekterzeugung Störungen zu lokalisieren und zu beheben oder die Effekterzeugung zielgerichtet zu verändern. Spitzen im langwelligen Bereich können z. B. auf unerwünschte Periodizitäten hinweisen, die mit entsprechenden Massnahmen vermieden werden können. **Figur 23(c)** zeigt das Spektrogramm des Grundgarns, d. h. der Kurve von Fig. 22(c). Allfällige Spitzen in diesem Spektrogramm geben gute Hinweise auf bestimmte Fehler im Spinnprozess oder in dem Spinnprozess vorangehenden Prozessstufen, etwa Exzentrizitäten bestimmter Walzen im Streckwerk. Diese Fehler können anhand der entsprechenden Wellenlängen im Spektrogramms von Fig. 23(c) lokalisiert und anschliessend behoben werden.

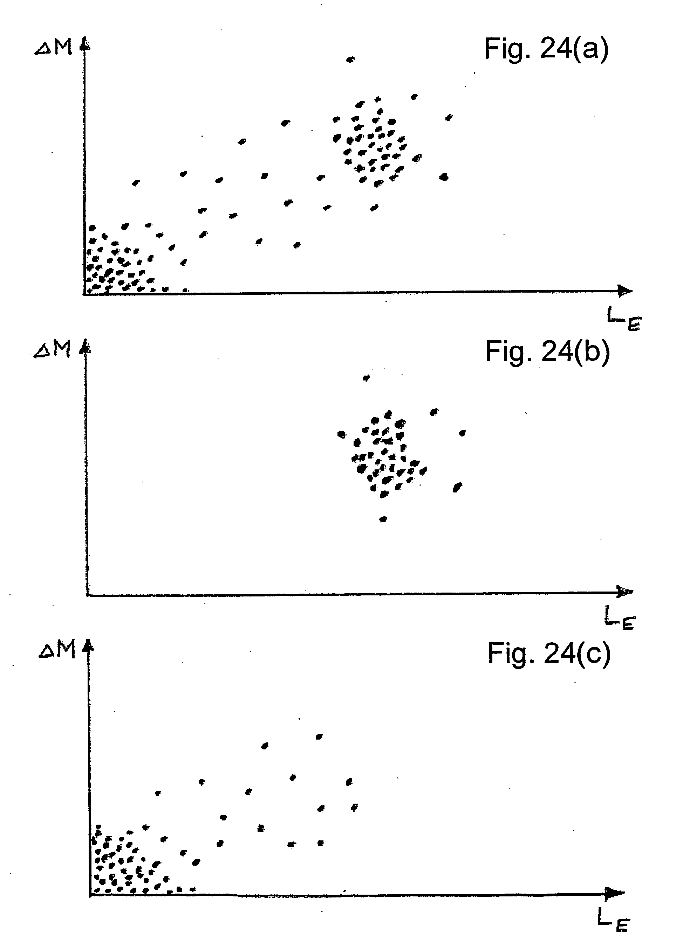

Ebenso können die Daten von Fig. 22 in einem Scatterplot dargestellt werden, analog zu Fig. 6. Auch diese Darstellungsart kann sehr nützlich sein, um zwischen Effekten und Grundgarn zu unterscheiden. Ein schematisches Beispiel ist in **Figur 24** angegeben. **Figur 24(a)** zeigt einen Scatterplot der ursprünglichen Messwerte der Kurve von Fig. 22(a). Hier vermischen sich drei Phänomene:
- die in jedem Garn auftretenden, wenig störenden Dickstellen,
- Effekte und
- irrtümlicherweise als Effekte ausgewertete Dickstellen.

**Figur 24(b)** zeigt einen Scatterplot der Effekte allein, wie sie in Fig. 22(b) dargestellt sind. Insbesondere dann, wenn das anlässlich von Fig. 21 beschriebene robuste Verfahren zur Effekterkennung zur Anwendung gelangt, wird dieser Scatterplot keine Punkte aufweisen, die unwerwünschterweise von Imperfektionen wie Dickstellen herrühren. Vielmehr enthält er nur wirkliche Effekte. **Figur 24(c)** zeigt einen Scatterplot des Grundgarns von Fig. 22(c). Die darin eingezeichneten Punkte stehen nicht für Effekte, sondern für Imperfektionen wie Dickstellen. Der Scatterplot von Fig. 24(c) kann Aufschluss geben über den verwendeten Garnherstellungsprozess.

Analog können auch andere Darstellungsarten wie z. B. die Histogramme von Fig. 5 getrennt für die effektbezogenen Daten einerseits und die grundgarnbezogenen Daten andererseits zum Einsatz kommen.

Es kann im erfindungsgemässen Verfahren von Vorteil sein, die Messwerte, wie sie etwa in Figur 2 oder 4 dargestellt sind, nach bestimmten Filterkriterien zu filtern. Solche Filterkriterien können bspw. folgende sein:
- Garnimperfektionen wie Nissen, Dick- und/oder Dünnstellen. So ist es etwa aus der CH-678'173 A5 oder der US-5,537,811 A bekannt, zum Einstellen der Reinigungsgrenze eines elektronischen Garnreinigers mögliche Garnfehler in einer Tabelle nach Art eines Koordinatensystems anzuordnen. Die Abszisse des Koordinatensystems stellt die Fehlermasse und die Ordinate die Fehlerlänge dar. In dieses Koordinatensystem werden eine obere und eine untere Reinigungsgrenze gelegt. Nissen und Dickstellen oberhalb der oberen Reinigungsgrenze und Dünnstellen unterhalb der unteren Reinigungsgrenze werden aus dem Garn automatisch entfernt. In analoger Weise kann auch beim erfindungsgemässen Verfahren vorgegangen werden, indem mindestens eine "Reinigungsgrenze" definiert wird. Dick- bzw. Dünnstellen innerhalb dieser Grenze werden herausgefiltert und nur Effekte ausserhalb dieser Grenze werden weiter ausgewertet und/oder grafisch dargestellt.
- Effektcharakteristiken. So können z. B. Effekte herausgefiltert werden, die eine bestimmte Effektlänge L_{E} unter- oder überschreiten, die eine bestimmte Effektgesamtmasse M_{E} unter- oder überschreiten, die eine bestimmte Effektform (vgl. Fig. 15) aufweisen oder nicht aufweisen, etc. Nur die verbleibenden, nicht herausgefilterten Effekte werden weiter ausgewertet und/oder grafisch dargestellt. Es ist auch möglich, mehrere verschiedene Filter vorzusehen, so dass bspw. mit einem ersten Filter nur kurze Effekte und mit einem zweiten Filter nur lange Effekte ausgewertet und/oder grafisch dargestellt werden können.

Vorzugsweise erlaubt das erfindungsgemässe Verfahren eine interaktive Eingabe bestimmter Parameter durch eine Bedienungsperson. Solche einzugebende Parameter können etwa Filterparameter für die oben diskutierten Filter sein. Auch Grundlagen für die Auswertung können als Parameter eingegeben werden, so z. B. eine vorgegebene Stegmasse M_{S}.

Es kann vorteilhaft sein, im erfindungsgemässen Verfahren eine Schnittstelle zur Ausgabe von Daten, die im Verfahren gewonnen wurden, vorzusehen. Solche Daten können z. B. die oben diskutierten Effektgarnparameter sein, die an eine Simulationssoftware übergeben werden. Die Simulationssoftware kann daraus eine Simulation des untersuchten Garns und eines aus dem Garn gewebten oder gestrickten Flächengebildes sein. Eine solche auf Auswertedaten basierende Simulation ist, verglichen mit einer auf Messdaten basierenden Simulation, verhältnismässig schnell und einfach.

Selbstverständlich ist die vorliegende Erfindung nicht auf die oben diskutierten Ausführungsformen beschränkt. Bei Kenntnis der Erfindung wird der Fachmann weitere Varianten herleiten können, die auch zum Gegenstand der vorliegenden Erfindung gehören. Einzelne oben beschriebene Merkmale des erfindungsgemässen Verfahrens, insbesondere die Auswertealgorithmen für die einzelnen Effektgarnparameter, können auch losgelöst von der erfindungsgemässen grafischen Darstellung Anwendung finden. Obwohl sich die vorliegende Beschreibung auf das Beispiel der kapazitiven Messung der Garnmasse konzentriert, ist die Erfindung nicht auf dieses Abtastprinzip beschränkt. Vielmehr können beim erfindungsgemässen Verfahren auch andere Abtastprinzipien - möglicherweise mit anderen Messgrössen - zum Einsatz kommen, z. B. die optische Messung des Garndurchmessers. Auch Kombinationen verschiedener Abtastprinzipien sind möglich.

### BEZUGSZEICHENLISTE

- 1: Vorrichtung
- 2: Abtasteinheit
- 23: erste Datenleitung
- 3: Auswerteeinheit
- 34: zweite Datenleitung
- 4: Ausgabeeinheit
- 5: Eingabeeinheit
- 9: Effektgarn
- 91, 91': Steg
- 92, 92': Effekt
- 93,94: Effektflanken
- 95: Effektdach
- 100: Messkurve
- 101: Grundpegel
- 102, 102': Signalspitze
- 103, 103', 103*: Effektbeginn
- 104, 104*: Effektende
- 105: lokales Maximum einer Spitze
- 106: Dünnstelle neben Effekt
- 111-115: Klassen von Effekten, Effektpopulationen
- 111.1-113.1: Teilflächen des Scatterplots
- 121: lokales Maximum, das zu Stegen gehört
- 122: lokales Maximum, das zu Effekten gehört
- 131: Verteilung im Spektrogramm
- 132: Maximum der Verteilung 121
- 133: Spitze im Spektrogramm
- A: Fläche unter der Messkurve
- B: Farbe Blau
- H: Häufigkeit eines Messwertes
- I_{M}: Massenintervall
- L: Periodenlänge
- L_{E}: Effektlänge
- L_{S}: Steglänge
- L_{E} + L_{S}: Effektabstand
- M: Masse pro Längeneinheit
- M_{E}: Effektgesamtmasse
- M_{M}: Mittelwert der gemessenen Masse pro Längeneinheit
- M_{S}: Stegmasse pro Längeneinheit
- M_{T}: Schwellenwert
- R: Farbe Rot
- x: Längskoordinate

- ΔM: Massenzunahme eines Effektes

## Patentansprüche

1. Verfahren zur Charakterisierung von Effektgarn (9), wobei
mindestens eine Eigenschaft des Effektgarns (9) entlang der Längsrichtung (x) des Effektgarns (9) abgetastet wird,
Werte der Abtastung ausgewertet werden und
Resultate der Auswertung ausgegeben werden,
**dadurch gekennzeichnet,**
**dass** die Auswertung eine Glättung oder Idealisierung der Abtastwerte beinhaltet, und die Glättung oder Idealisierung durch Annäherung der aus den Abtastwerten gebildeten Messkurve durch gerade Streckenabschnitte erfolgt.

2. Verfahren nach Anspruch 1, wobei Effekte (92, 92') des Effektgarns (9) als Trapeze, Dreiecke oder Rechtecke angenähert werden.

3. Verfahren nach Anspruch 2, wobei jedes Trapez, Dreieck oder Rechteck derart gewählt wird, dass seine Fläche gleich der Fläche unter einer aus den ursprünglichen Abtastwerten erzeugten Messkurve (M(x)) ist.

4. Verfahren nach Anspruch 2 oder 3, wobei die Effektlänge (L_{E}) als Basislänge des Trapezes, Dreiecks oder Rechtecks definiert wird.

5. Verfahren nach einem der vorangehenden Ansprüche, wobei Stege (91, 91') des Effektgarns (9) durch waagrechte Strecken angenähert werden, die alle auf der Höhe einer zuvor ermittelten Stegmasse (M_{S}) liegen.

6. Verfahren nach einem der vorangehenden Ansprüche, wobei die Auswertung eine Verknüpfung der geglätteten oder idealisierten Abtastwerte mit den ursprünglichen Abtastwerten beinhaltet.

7. Verfahren nach Anspruch 6, wobei die Verknüpfung eine Differenzbildung oder eine Quotientenbildung ist.

8. Verfahren nach Anspruch 6 oder 7, wobei die geglätteten oder idealisierten Abtastwerte und die aus der Verknüpfung hervorgegangenen Daten separat ausgewertet werden, um Information über die Effekte (102, 102') einerseits bzw. über das Grundgarn andererseits zu erhalten.

9. Verfahren nach einem der vorangehenden Ansprüche, wobei die Auswertung eine Filterung der Abtastwerte beinhaltet.

10. Verfahren nach Anspruch 9, wobei als Filterkriterien Garnimperfektionen und/oder Effektcharakteristiken gewählt werden.

11. Verfahren nach einem der vorangehenden Ansprüche, wobei mindestens ein für die Auswertung benötigter Parameter interaktiv eingebbar ist.

12. Verfahren nach einem der vorangehenden Ansprüche, wobei mindestens ein Resultat der Auswertung in Form einer grafischen Darstellung ausgegeben wird.

13. Verfahren nach Anspruch 12 und einem der Ansprüche 6-7, wobei eine eigene grafische Darstellung für die geglätteten oder idealisierten Abtastwerte und/oder eine eigene grafische Darstellung für die aus der Verknüpfung hervorgegangenen Daten ausgegeben wird.

14. Verfahren nach Anspruch 12 oder 13, wobei die grafische Darstellung aus der Gruppe von Diagrammen ausgewählt wird, welche eine Aufzeichnung einer abgetasteten Effektgarneigenschaft gegenüber der Position (x) auf dem Effektgarn (9) oder gegenüber der Zeit, ein Histogramm, vorzugsweise in Form eines zwei- oder dreidimensionalen Säulen- oder Balkendiagramms, ein Scatterplot, eine Klassiermatrix, eine Fläche in dreidimensionaler oder zweidimensionaler Darstellung, ein Säulendiagramm, ein Balkendiagramm, ein Kreisdiagramm, ein Kuchendiagramm, eine Tabelle und ein Spektrogramm umfasst.

15. Verfahren nach einem der Ansprüche 12-14, wobei anhand der grafischen Darstellung verschiedene Klassen (111-113) von Effekten (92) identifiziert werden.

16. Verfahren nach einem der vorangehenden Ansprüche, wobei bei der Auswertung aus den Werten der Abtastung eine Messkurve (M(x)) erzeugt und eine Fläche (A(x)) unter der Messkurve (M(x)) berechnet wird.

17. Verfahren nach Anspruch 16, wobei die Fläche (A(x)) zwischen der Messkurve (M(x)) und einer zuvor bestimmten Stegmasse (M_{S}) oder einem zuvor bestimmten Stegdurchmesser berechnet wird.

18. Verfahren nach einem der vorangehenden Ansprüche, wobei die mindestens eine abgetastete Eigenschaft eine Masse (M) und/oder ein Durchmesser des Effektgarns (9) ist.

19. Verfahren nach einem der vorangehenden Ansprüche, wobei Resultate der Auswertung aus der Gruppe von Effektgarnparametern ausgewählt werden, welche eine Stegmasse (M_{S}), einen Stegdurchmesser, eine Steglänge (L_{S}), eine Massenzunahme (ΔM) eines Effektes (92), eine Effektdurchmesserzunahme, einen Effektdurchmesser, eine Effektlänge (L_{E}), eine Effektgesamtmasse (M_{E}), eine mittlere Garnnummer, eine Anzahl Effekte pro Längeneinheit, eine Rapportlänge, eine Unterraportlänge, eine Form und eine Farbe umfasst.

20. Verfahren nach Anspruch 19, wobei jedem Effekt (92, 92') eine Laufnummer zugeordnet und die Laufnummer zusammen mit den Parametern des zugehörigen Effektes (92) gespeichert wird.

21. Verfahren nach Anspruch 19 oder 20, wobei Resultate der Auswertung Minima, Maxima, arithmetische Mittel und/oder Standardabweichungen der Effektgarnparameter und/oder die Anzahl Effekte (92) pro Garnlänge sind.

## Claims

1. A method for the characterization of fancy yarn (9), wherein
at least one characteristic of the fancy yarn (9) is scanned along the longitudinal direction (x) of the fancy yarn (9),
values of the scanning are evaluated and
results of the evaluation are outputted,
**characterized in that**
the evaluation includes a smoothing or idealization of the scanning values, and
the smoothing or idealization is effected by an approximation of the measurement curve produced from the scanning values by straight stretch sections.

2. The method according to claim 1, wherein slubs (92, 92') of the fancy yarn (9) are approximated as trapeziums, triangles or rectangles.

3. The method according to claim 2, wherein each trapezium, triangle or rectangle is selected in a manner such that its area is equal to the area below a measurement curve (M(x)) produced from the original scanning values.

4. The method according to claim 2 or 3, wherein the slub length (L_{E}) is defined as the base length of the trapezium, triangle or rectangle.

5. The method according to one of the preceding claims, wherein base yarn sections (91, 91') of the fancy yarn (9) are approximated by horizontal stretches which all lie at the height of a previously determined base yarn mass (M_{S}).

6. The method according to one of the preceding claims, wherein the evaluation includes a linking of the smoothed or idealized scanning values with the original scanning values.

7. The method according to claim 6, wherein the linking is a difference formation or a quotient formation.

8. The method according to claim 6 or 7, wherein the smoothed or idealized scanning values and the data which have come from the linking are evaluated separately, in order to obtain on the one hand information on the slubs (102, 102') and on the other hand on the virtual base yarn.

9. The method according to one of the preceding claims, wherein the evaluation includes a filtering of the scanning values.

10. The method according to claim 9, wherein yarn imperfections and/or slub characteristics are selected as filtering criteria.

11. The method according to one of the preceding claims, wherein at least one parameter required for the evaluation may be inputted in an interactive manner.

12. The method according to one of the preceding claims, wherein at least one result of the evaluation is outputted in the form of a graphic representation.

13. The method according to claim 12 and one of the claims 6-7, wherein an individual graphic representation for the smoothed or idealized scanning values and/or an individual graphic representation for the data which has come from the linking is outputted.

14. The method according to claim 12 or 13, wherein the graphic representation is selected from the group of diagrams which comprises a recording of a scanned fancy yarn characteristic with respect to the position (x) on the fancy yarn (9) or with respect to time, a histogram, preferably in the form of a two- or three-dimensional column chart or bar chart, a scatter diagram, a classification matrix, a surface in a three-dimensional or two-dimensional representation, a column chart, a bar chart, a circular chart, a pie chart, a table and a spectrogram.

15. The method according to one of the claims 12-14, wherein different classes (111-113) of slubs (92) are identified by way of the graphic representation.

16. The method according to one of the preceding claims, wherein on evaluation, a measurement curve (M(x)) is produced from the values of the scanning, and an area (A(x)) below the measurement curve (M(x)) is computed.

17. The method according to claim 16, wherein the area (A(x)) between the measurement curve (M(x)) and a previously determined base yarn mass (M_{S}) or a previously determined base yarn diameter is computed.

18. The method according to one of the preceding claims, wherein the at least one scanned characteristic is a mass (M) and/or a diameter of the fancy yarn (9).

19. The method according to one of the preceding claims, wherein results of the evaluation are selected from the group of fancy yarn parameters which includes a base yarn mass (M_{S}), a base yarn diameter, a slub distance (L_{S}), a mass increase (ΔM) of a slub (92), and slub diameter increase, a slub diameter, and slub length (L_{E}), a slub total mass (M_{E}), an average yarn count, a number of slubs per length unit, a pattern length, a sub-pattern length, a shape and a color.

20. The method according to claim 19, wherein a running number is allocated to each slub (92, 92'), and the running number is stored together with the parameters of the associated slub (92).

21. The method according to claim 19 or 20, wherein results of the evaluation are minima, maxima, arithmetic means and/or standard deviations of the fancy yarn parameters and/or the number of slubs (92) per yarn length.

## Revendications

1. Procédé pour la caractérisation d'un filé à effets (9), dans lequel
au moins une propriété du filé à effets (9) est détectée dans le sens longitudinal (x) du filé à effets (9) ;
des valeurs de détection sont analysées ;
des résultats de l'analyse sont émis en sortie,
**caractérisé en ce que**
l'analyse comprend un lissage ou une idéalisation des valeurs de détection et
le lissage ou l'idéalisation est effectué par approximation de la courbe de mesure formée à partir des valeurs de détection à l'aide de segments de droite.

2. Procédé selon la revendication 1, dans lequel des effets (92, 92') du filé à effets (9) sont approchés sous forme de trapèzes, de triangles ou de rectangles.

3. Procédé selon la revendication 2, dans lequel chaque trapèze, triangle ou rectangle est choisi de telle façon sur son aire soit égale à l'aire sous une courbe de mesure (M(x)) générée à partir des valeurs de détection d'origine.

4. Procédé selon la revendication 2 ou 3, dans lequel la longueur de l'effet (L_{E}) est définie comme longueur de la base du trapèze, du triangle ou du rectangle.

5. Procédé selon l'une des revendications précédentes, dans lequel des sections de filé entre deux effets (91, 91') du filé à effets (9) sont approchées par des longueurs horizontales qui se trouvent toutes au niveau d'une masse de la section de filé entre deux effets (M_{S}) déterminée au préalable.

6. Procédé selon l'une des revendications, dans lequel l'analyse inclut une association des valeurs de détection lissées ou idéalisées avec les valeurs de détection d'origine.

7. Procédé selon la revendication 6, dans lequel l'association est un calcul de différence ou un calcul de quotient.

8. Procédé selon la revendication 6 ou 7, dans lequel les valeurs de détection lissées ou idéalisées et les données issues de la liaison sont analysées séparément afin d'obtenir des informations sur les effets (102, 102') d'une part et sur le filé de base d'autre part.

9. Procédé selon l'une des revendications précédentes, dans lequel l'analyse comprend un filtrage des valeurs de détection.

10. Procédé selon la revendication 9, dans lequel les critères de filtrage choisis sont des imperfections du filé et/ou des caractéristiques de l'effet.

11. Procédé selon l'une des revendications précédentes, dans lequel au moins un paramètre nécessaire pour l'analyse peut être saisi de façon interactive.

12. Procédé selon l'une des revendications précédentes, dans lequel au moins un résultat de l'analyse est émis sous la forme d'une représentation graphique.

13. Procédé selon la revendication 12 et l'une des revendications 6-7, dans lequel une représentation graphique propre est émise pour les valeurs de détection lissées ou idéalisées et/ou une représentation graphique propre pour les données issues de l'association.

14. Procédé selon la revendication 12 ou 13, dans lequel la représentation graphique est choisie dans le groupe de diagrammes comprenant un enregistrement d'une propriété du filé à effets détectée par rapport à la position (x) sur le filé à effets (9) ou par rapport au temps, un histogramme, de préférence sous la forme d'un diagramme à colonnes ou d'un diagramme à bâtons à deux ou trois dimensions, un diagramme de dispersion, une matrice de classification, une aire dans une représentation à trois ou deux dimensions, un diagramme à colonnes, un diagramme à bâtons, un diagramme circulaire, un diagramme camembert, un tableau et un spectrogramme.

15. Procédé selon l'une des revendications 12 à 14, dans lequel différentes classes (111-113) d'effets (92) sont identifiées à partir de la représentation graphique.

16. Procédé selon l'une des revendications précédentes, dans lequel lors de l'analyse, une courbe de mesure (M(x)) est générée à partir des valeurs de détection et une aire (A(x)) sous la courbe de mesure (M(x)) est calculée.

17. Procédé selon la revendication 16, dans lequel l'aire (A(x)) entre la courbe de mesure (M(x)) et une masse de la section de filé entre deux effets (M_{S}) déterminée précédemment ou un diamètre de la section de filé entre deux effets déterminé précédemment est calculée.

18. Procédé selon l'une des revendications précédentes, dans lequel l'au moins une propriété détectée est une masse (M) et/ou un diamètre du filé à effets (9).

19. Procédé selon l'une des revendications précédentes, dans lequel des résultats de l'analyse sont choisis dans le groupe de paramètres du filé à effets qui comprend une masse (M_{S}) de la section de filé entre deux effets, un diamètre de la section de filé entre deux effets, une longueur (L_{S}) de la section de filé entre deux effets, une augmentation de masse (ΔM) d'un effet (92), une augmentation de diamètre de l'effet, un diamètre de l'effet, une longueur de l'effet (L_{E}), une masse totale de l'effet (M_{E}), un nombre de fils moyen, un nombre d'effets par unité de longueur, une longueur de motif, une longueur de sous-motif, une forme et une couleur.

20. Procédé selon la revendication 19, dans lequel chaque effet (92, 92') est associé à un numéro d'ordre et le numéro d'ordre est enregistré avec les paramètres de l'effet (92).

21. Procédé selon l'une des revendications 19 ou 20, dans lequel les résultats de l'analyse sont des minima, des maxima, des moyennes arithmétiques et/ou des écarts-types des paramètres du filé à effets et/ou le nombre d'effets (92) par longueur de fil.
